# EUROPEAN PATENT APPLICATION

(11) **EP 3 799 883 A1**
(43) Date of publication of application: **07.04.2021**
(21) Application number: 20206493.7
(22) Date of filing: 23.11.2017
(51) Int. Cl.: A61K 39/015, A61K 47/69, A61K 39/095

(54) **NOMV-ANTIGEN CONJUGATED COMPOUNDS AND USE THEREOF**

(30) Priority: 25.11.2016 GB 201619949; 27.07.2017 GB 201712101
(62) Divisional of application: 17816489.3
(71) Applicant: GlaxoSmithKline Biologicals S.A., 1330 Rixensart (BE)
(72) Inventor: DI BENEDETTO, Roberta, 53100 Siena (IT); MICOLI, Francesca, 53100 Siena (IT); SAUL, Allan James, 53100 Siena (IT)
(74) Representative: Klaczynska, Sarah Elizabeth

(57) **Abstract**

The provided technology is in the field of conjugating native, non-detergent extracted, outer membrane vesicles (nOMV) to antigens to form nOMV-linker-antigen conjugates, which are particularly useful for immunogenic compositions and immunisation; processes for the preparation and use of such conjugates is also provided.

## Description

### TECHNICAL FIELD

This invention is in the field of conjugating non-detergent extracted, native outer membrane vesicles (nOMVs) to antigens, via a divalent linker, to form nOMV-antigen conjugates. The conjugates are useful for immunisation.

### BACKGROUND ART

Conjugation of antigens to carriers is an established procedure for improving immunogenicity, particularly for polysaccharides. For instance, bacterial capsular polysaccharides (CPS) are naturally T-cell independent antigens which give rise to an immune response that lacks several important properties. Conjugation to a carrier converts these saccharides to T-cell dependent antigens which can then produce an immunological memory effect, and also elicit protective immune responses in young children.

One known carrier in such conjugates is the 'OMPC' outer membrane protein complex formed from *N.meningitidis* vesicles (*e.g.* see EP-0467714), which has been included as the carrier in approved *H.influenzae* B conjugate vaccines. OMPC has also been used as the carrier in protein conjugates. For example, Wu et al. (PNAS USA 2006; 103(48): 18243-18248) report that conjugation of Pfs25H to OMPC resulted in a Pfs25H-OMPC conjugate vaccine that was >1,000 times more potent in generating anti-Pfs25H ELISA reactivity in mice than a similar dose of Pfs25H alone.

Conjugation of OMPC to Pfs25H protein can be achieved by reacting maleimide-activated Pfs25H with thiolated outer membrane proteins within OMPC (see e.g. WO2006/124712), as shown in Scheme 1.

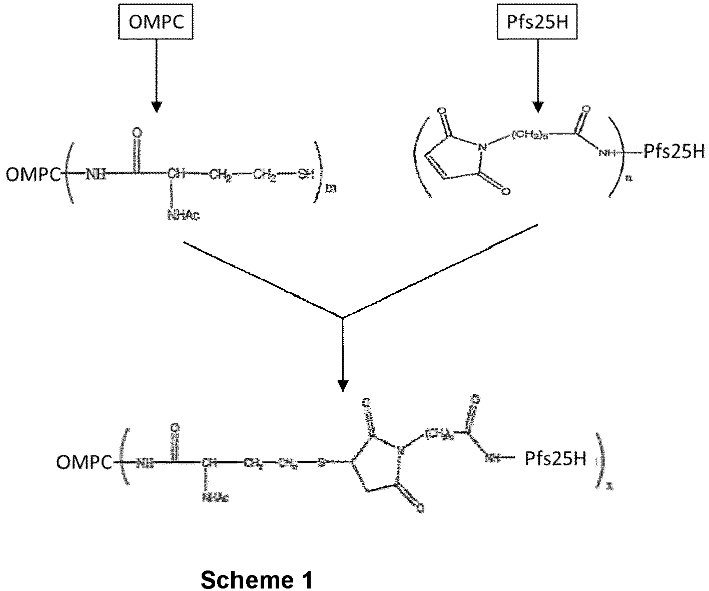

As shown in the above Scheme 1 and according to general procedures of the prior art, the conjugation methods and conjugates thereof contemplate the use of a divalent heterobifunctional linker, *i.e.* a linker moiety having the terminal ends bearing different functional groups. This is mainly to avoid cross linking of the vesicle-linker intermediate with another vesicle particle rather than with the selected antigen. In practice, according to the prior art, the different ends of the linker are selected depending on the reactive groups on the vesicle and the selected antigen involved in the process, in order to have a selective reaction with the intended part, namely the vesicle on one side and the antigen on the other end side. These methodologies, however, suffer of some drawbacks, mainly related to the selection and functionalization of the linkers, thus posing some limitation on the choice of the vesicle and antigen to be coupled together. The Applicant has now found that when native, non-detergent extracted Outer Membrane Vesicles (nOMVs) are used as starting vesicles it is possible to use a divalent linker suitable for the connection with a nOMV surface protein on one side and with a selected antigen on the other end, thus providing a final conjugate that still presents the immunogenic activity of both the nOMV and the antigen. Surprisingly, even when the linker used in the present invention shows identical terminal functional groups, the conjugation of the nOMV with the selected antigen is achieved substantially without the formation of vesicle aggregates or side products, detrimental for the conjugation reaction.

### SUMMARY OF THE INVENTION

In a first aspect, the invention refers to an immunogenic nOMV-linker-antigen conjugate comprising a native Outer Membrane Vesicle (nOMV) obtained by a detergent free process, having at least a surface protein residue connected to at least a selected foreign antigen by a divalent linker.

In a further aspect, the invention refers to a process for preparing said conjugate, comprising the steps of:
i) reacting at least a nOMV surface protein residue with the first terminal portion of a divalent linker to obtain a nOMV-linker intermediate, and
ii) reacting said nOMV-linker intermediate with at least one selected foreign antigen via the second terminal portion of the divalent linker, thus obtaining the nOMV-linker-antigen conjugate of the invention.

According to an embodiment, the linker is a divalent homobifunctional linker, *i.e.* having the same terminal functionalities. In a still preferred embodiment, the linker is a divalent heterobifunctional linker, *i.e.* having different terminal functionalities.

In a further aspect, the invention refers to the nOMV-linker intermediate obtained (or obtainable) by the above indicated step i), and its use for the preparation of nOMV-linker-antigen conjugate of the invention.

In one additional aspect, the invention also refers to the above nOMV-linker-antigen conjugate for use as an immunogenic compound, particularly for the preparation of an immunogenic composition or vaccine.

In a further aspect, the immunogenic conjugates according to the invention comprise a nOMV having at least a surface protein moiety connected to a first antigen by a divalent linker, wherein said first antigen is further connected to a second different antigen.

In a still further aspect, the immunogenic conjugates of the invention comprises a nOMV having at least a surface protein moiety connected to a first antigen by a divalent linker, and at least another surface protein moiety connected to a second different antigen by a divalent linker, wherein the divalent linker connecting the first antigen and the divalent linker connecting the second agent are independently the same or different to each other.

In a still further aspect, the invention refers to an immunogenic composition or a vaccine, comprising the above indicated conjugate and at least one pharmaceutically acceptable carrier or adjuvant; and to a method for raising an immune response in a vertebrate, comprising the administration of said composition or vaccine.

In a further aspect, the invention also refers to the use of nOMV for the preparation of immunogenic polyvalent nOMV-linker-antigen conjugates.

### BRIEF DESCRIPTION OF THE DRAWINGS

Figure 1 shows anti-CSP (1A) and anti-OAg (1B) IgG titers after immunisation with CSP or its fragment (NANP)₃ conjugated to S. Typhimurium nOMV by SH-malemido chemistry, compared to CSP physically mixed with said nOMV (formulated with no adjuvant). CD1 female mice, 5 weeks old (8 per group) were subcutaneously immunised at days 0 and 28 with 2 µg CSP/(NANP)₃ per dose. Titres were measured at days 0, 14, 28 and 42.
Figure 2: Anti-Pfs25 IgG response induced in mice (200 µL per dose SC injected at days 0 and 28, bleeds at days 0, 14, 27 and 42) by nOMV conjugates of the invention produced by conjugating S. Typhimurium nOMV with Pfs25 antigen, via BS3 linker.
Figure 3: Anti-fHbp IgG response induced in mice (200 µL per dose SC injected at days 0 and 28, bleeds at days 0, 14, 27 and 42) by nOMV conjugates of the invention produced by conjugating S. Typhimurium nOMV with fHbp antigen, via BS3 linker.
Figure 4: Anti-MenA IgG response induced in mice by nOMV conjugates of the invention produced by conjugating MenB nOMV with MenA antigen, via SIDEA linker, compared with MenA-MenC bivalent MenBGMMA conjugate, GMMA, CRM conjugate and mixture of MenA and GMMA (not conjugated).
Figure 5: Anti-MenC IgG response induced in mice by nOMV conjugates of the invention produced by conjugating MenB nOMV with MenC antigen, via SIDEA linker, compared with MenA-MenC bivalent MenBGMMA conjugate, GMMA, CRM conjugate and mixture of MenC and GMMA (not conjugated).

### DETAILED DESCRIPTION OF THE INVENTION

To facilitate an understanding of the present invention, a number of terms and phrases are defined below. Art-recognized synonyms or alternatives of the following terms and phrases (including past, present, etc. tenses), even if not specifically described, are contemplated.

As used in the present disclosure and claims, the singular forms "a," "an," and "the" include plural forms unless the context clearly dictates otherwise; *i.e.,* "a" means "one or more" unless indicated otherwise.

The terms "about" or "approximately" mean roughly, around, or in the regions of. The terms "about" or "approximately" further mean within an acceptable contextual error range for the particular value as determined by one of ordinary skill in the art, which will depend in part on how the value is measured or determined, *i.e.* the limitations of the measurement system or the degree of precision required for a particular purpose, e.g. the amount of a nutrient within a feeding formulation. When the terms "about" or "approximately" are used in conjunction with a numerical range, it modifies that range by extending the boundaries above and below the numerical values set forth. For example "between about 0.2 and 5.0 mg/ml" means the boundaries of the numerical range extend below 0.2 and above 5.0 so that the particular value in question achieves the same functional result as within the range. For example, "about" and "approximately" can mean within 1 or more than 1 standard deviation as per the practice in the art. Alternatively, "about" and "approximately" can mean a range of up to 20%, preferably up to 10%, more preferably up to 5%, and more preferably up to 1% of a given value.

The term "and/or" as used in a phrase such as "A and/or B" is intended to include "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

Unless specified otherwise, all of the designations "A%-B%," "A-B%," "A% to B%," "A to B%," "A%-B," "A% to B" are given their ordinary and customary meaning. In some embodiments, these designations are synonyms.

The terms "substantially" or "substantial" mean that the condition described or claimed functions in all important aspects as the standard described. Thus, "substantially free" is meant to encompass conditions that function in all important aspects as free conditions, even if the numerical values indicate the presence of some impurities or substances. "Substantial" generally means a value greater than 90%, preferably greater than 95%, most preferably greater than 99%. Where particular values are used in the specification and in the claims, unless otherwise stated, the term "substantially" means with an acceptable error range for the particular value.

An "effective amount" means an amount sufficient to cause the referenced effect or outcome. An "effective amount" can be determined empirically and in a routine manner using known techniques in relation to the stated purpose.

As used herein, "heterologous" means the two or more referenced molecules or structures are derived from a different organism. For example, for a nOMV, a heterologous antigen is one that is derived from a different organism than the nOMV vesicle to which it is appended. "Homologous" as used herein means the two or more referenced molecules or structures are derived from the same organism.

As used herein, "foreign" means the two or more referenced molecules or structures are not naturally associated with each other. For example, a selected antigen that is herein intended to be "foreign to" a nOMV surface saccharide herein means the antigen is not naturally or innately conjugated to the surface saccharide and is, therefore, not naturally conjugated to the nOMV molecule even though the antigen and the saccharide (or nOMV molecule) may originate from the same organism. In this way, a foreign antigen is not necessarily a heterologous antigen but a heterologous antigen is a foreign antigen.

"Sequence identity" can be determined by the Smith-Waterman homology search algorithm as implemented in the MPSRCH program (Oxford Molecular), using an affine gap search with parameters gap open penalty=12 and gap extension penalty=1, but is preferably determined by the Needleman-Wunsch global alignment algorithm (see *e.g.* Rubin (2000) Pediatric. Clin. North Am. 47:269-285), using default parameters (*e.g.* with Gap opening penalty = 10.0, and with Gap extension penalty = 0.5, using the EBLOSUM62 scoring matrix). This algorithm is conveniently implemented in the needle tool in the EMBOSS package. Where the application refers to sequence identity to a particular SEQ ID, the identity is intended to be calculated over the entire length of that SEQ ID.

The term "% w/w" indicates the weight percentage of a given compound, over a different compound or over the whole content of a composition, as indicated.

Analogously, the term "% v/v" indicates the volume percentage of a given compound, over a different compound or over the whole content of a composition, as indicated.

As herein used, the term "saccharide (or sugar) moiety" comprises in its meaning mono saccharides, as well as polysaccharide units. It will be appreciated that saccharide moieties can exist in open and closed (ring) form and that, while closed forms are shown in structural formulae herein, open forms are also encompassed by the invention. Similarly, it will be appreciated that saccharide moieties can exist in pyranose and furanose forms and that, while pyranose forms are shown in structural formulae herein, furanose forms are also encompassed. Different anomeric forms of saccharide moieties are also encompassed.

The term "oligosaccharide" comprises in its meaning polysaccharides having from 3 to 10 monosaccharide units, as generally known in the art (see *e.g.* https://en.wikipedia.org/wiki/Oligosaccharide).

Unless otherwise provided, the term "polypeptide" refers to polypeptides of any length capable to act as a selected antigen. The amino acid polymer forming the polypeptide of the invention, may be linear or branched, it may comprise modified amino acids, and it may be interrupted by non-amino acids. The term also encompasses an amino acid polymer that has been modified naturally or by intervention; for example, disulfide bond formation, glycosylation, lipidation, acetylation, phosphorylation, or any other manipulation or modification, such as conjugation with a labelling component. Also included within the definition are, for example, polypeptides containing one or more analogues of an amino acid (including, for example, unnatural amino acids, etc.), as well as other modifications known in the art. Polypeptides can occur as single chains or associated chains.

"Average molecular weight" is intended to indicate the average molecular weight obtained by the ordinary arithmetic mean or average of the molecular masses of the individual component, *e.g.* amino acids in case of polypeptide conjugates.

The term "-OAg" (O-antigen) is used within the present invention to indicate an antigen functionality present in the lipopolysaccharides (LPS) or lipooligosaccharides (LOS) on the surface of the considered nOMV. In more details, the LPS are generally formed by three different portions, known as: lipidA (responsible for the toxicity of LPS), core oligosaccharide and the -OAg chain, a repetitive glycan polymer and major contributor to the serological specificity of bacteria.

The term "capsular polysaccharides/saccharides" (CPSs) indicates those saccharides which can be found in the layer that lies outside the cell envelope of bacteria, thus being part of the outer envelope of the bacterial cell itself. CPSs are expressed on the outermost surface of a wide range of bacteria, and in some cases even in fungi.

The term "suspension" comprises in its meaning a liquid medium presenting some precipitates or particulates or flocculating particles, differently from a solution where the medium is substantially free of any solid particles.

Unless otherwise provided, the term "conjugation" indicates the connection or linkage of the subjected entities, particularly referred to the nOMV and the selected antigen moieties, via a divalent linker.

By "immunologically effective amount", it is meant that the administration of that amount to an individual, either in a single dose or as part of a series, is effective for treatment or prevention. This amount can vary depending upon the health and physical condition of the individual to be treated, age, the taxonomic group of individual to be treated (*e.g.* non-human primate, primate, etc.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

The term "nOMVs" herein indicates vesicles isolated from the medium and/or sheared from cells, and they are intact membrane vesicles not exposed to detergents or denaturating agents, *i.e.* non detergent extracted. The nOMVs of the invention present the outer membrane proteins (OMP) and/or lipopolysaccharide (LPS) in their native conformation and correct orientation in the natural membrane environment, and usually lack the cytoplasmatic components.

On the contrary, the term "OMV" or "dOMV" encompasses a variety of proteoliposomic vesicles obtained by disruption of the outer membrane of a Gram-negative bacterium typically by a detergent extraction process to form vesicles therefrom. Outer membrane protein complexes (*e.g.* OMPC from Neisseria meningitides) may be considered in such definition, since having three dimensional structure and composition similar to dOMV, and being isolated via detergent extraction procedures (see *e.g.* EP0467714, US4271147, US4459286 and US4830852). The detergent extraction process removes LPS and phospholipids, together with immunoprotective lipoproteins. Such removal changes the native vesicle structure and promotes aggregation. The aggregation may lead to consequent issues in terms of process development (yield, consistency of production and stability). Differently from nOMVs, characterized by defined homogeneous size distribution (typically in the range 20-250 nm, measured by Dynamic Light Scattering DLS technique), the dOMVs have an undefined heterogeneous size distribution (usually in the range 550-5500 nm as measured by Dynamic Light Scattering DLS technique) caused by detergent-induced vesicle aggregation (see for a general reference, Vaccine 28, 2010, 4810). The detergent extraction process also causes contamination of OMV containing composition (e.g. vaccines) with cytoplasmic proteins as a result of bacterial cell lysis.

According to prior art methodologies, dOMVs and nOMVs may be analysed and described in terms of size, shape and overall appearance of impurities or contaminating non-OMV materials (like vesicle aggregates or detergent residues in case of dOMVs) using the Transmission Electron Microscopy (TEM). For detailed references regarding the differences between dOMVs and nOMVs see *e.g.* van de Waterbeemd et al. J. Prot. Res. 12(4) (2013) 1898-1908 "Quantitative Proteomics Reveals Distinct Differences in the Protein Content of Outer Membrane Vesicle Vaccines"; and J. Klimentova et al. Microbiological Research 170 (2015) 1-9 "Methods of isolation and purification of the outer membrane vesicles from gram-negative bacteria".

The term "divalent homobifunctional linker" or "homologous linker" indicates a linking unit presenting two terminal ends bearing the same functional group, and able to react with the nOMV protein on one side, and with the selected antigen on the other side, where nOMV protein and selected antigen are as herein below described in details.

Similarly, the term "divalent heterobifunctional linker" or "heterologous linker" indicates a linking unit presenting two terminal ends bearing different functional groups, and able to specifically react with the nOMV protein on one side, and with the selected antigen on the other side, where nOMV protein and selected antigen are as herein below described in detail.

The term "divalent C₁-C₁₀ alkyl or alkenyl group" comprises in its meaning a divalent satured or unsatured alky or alkenyl group having 1 to 10 carbon atoms, such as methylene, ethylene, vinyl, allyl and the like.

As above introduced, the invention refers to nOMV-linker-antigen conjugates obtained by covalently connecting at least a protein unit on the nOMV surface to one or more selected foreign antigen(s), via suitable divalent linker(s). In other terms, and according to one aspect, the invention refers to nOMV-linker-antigen conjugates obtained (or obtainable) by the process of the invention as herein below described in more details.

Of note, the present conjugates are endowed with a remarkable immunogenic activity, as further supported by the herein experimental part. In fact, as well as being capable of inducing an immune response against the conjugated antigen, the conjugates of the invention are also capable of inducing an immune response against the nOMV component, differently from dOMV-antigen conjugates of the art where the immune activity relies mainly on the antigen portion and not on the detergent extracted vesicle. On the contrary, according to the present invention, the conjugation of the selected antigen to nOMV protein component, does not significantly impact on the ability of the nOMV to induce its own immune response. Of note, when nOMV and the selected antigen(s) are from different sources, the conjugates of the invention may be useful for instance for the preparation of multi-valent immunogenic compositions or vaccines based on both the nOMV and the conjugated antigen(s) activity.

Even further, the present invention surprisingly shows that a homobifunctional linker can be used in the preparation of nOMV-linker-antigen conjugates, without incurring in the prior art problems related e.g. to vesicle cross reaction or aggregation. In practice, the starting nOMV is functionalized with the homobifunctional linker by reacting proper functional groups of at least one vesicle surface protein with one end of the linker. By that, a nOMV-linker intermediate is covalently formed, still having the other end of the linker available for the subsequent reaction with the selected antigen. Thus, the second end of the linker will react with the selected antigen, in a specific and selective way, leading to the final nOMV-linker-antigen conjugate, and substantially avoiding intermediate cross reactions or aggregations. As indicated in the Example 8a, the same reaction when carried out considering a dOMV as starting vesicle leads to the formation of vesicle-linker-vesicle aggregates, which are not suitable for a subsequent reaction with the selected antigen. Surprisingly it has now found that not only the use of nOMV as starting vesicle can overcome the aggregation problems of the prior art, but also it is now possible to use a variety of bifunctional linkers that lead to the preparation of a series of nOMV-linker-antigens conjugates according to the invention, retaining the immunogenic profile of the conjugated components.

The conjugates of the invention offer several advantages compared to unconjugated antigens, for example the ability to act as a bivalent or multivalent immunogenic agent or vaccine as discussed above, and improved immunogenicity over unconjugated antigens (see Examples 5 and 7). In addition, conjugates of the invention have several advantages over the vesicle-protein conjugates that have been used to date. Firstly, the nOMVs can be prepared with fewer steps compared to dOMV conjugates in which antigens were coupled to dOMV proteins, and in particular without requiring the expensive and time consuming step of protein derivatisation. Secondly, the production of nOMVs can be more reliable and convenient than the preparation of dOMVs by detergent extraction. Even further, unreacted antigen from the conjugation mixture can be recycled for use in a further conjugation step, improving the efficiency of production of the conjugates as exemplified in Example 3.

In a first aspect, the invention refers to a conjugate comprising one or more different selected foreign antigen(s), each connected to a protein residue of a non-detergent extracted native Outer Membrane Vesicle (nOMV), through a divalent linker. Of note, the nOMV can be collected and isolated substantially without the use of detergents, differently for instance from dOMVs of the prior art obtained via a deoxycholate extraction or using zwitterionic detergents like Empigen BB (see *e.g.* US4707543) or similar.

In more details, the nOMVs are naturally-occurring membrane vesicles that form spontaneously during bacterial growth and are released into culture medium. They can be obtained *e.g.* by culturing bacteria in broth culture medium, separating whole cells from the smaller nOMVs in the broth culture medium (*e.g.* by filtration or by low-speed centrifugation to pellet only the cells and not the smaller vesicles), and then collecting the nOMVs from the cell-depleted medium (*e.g.* by filtration, by differential precipitation or aggregation, by high-speed centrifugation to pellet the vesicles). Strains for use in production of nOMVs can generally be selected on the basis of the amount of nOMVs produced in culture. As above set forth, the present nOMVs are characterised by the fact of being collected and isolated following a detergent-free procedure. Preferably, the nOMV are released into the fermentation broth and are purified using a centrifugation and subsequent filtration step (for a general reference see e.g. Clin. Vaccine Immunol. 2016 Apr; 23(4): 304-314). Still preferably, the nOMV are released into the fermentation broth and are purified using the following two consecutive Tangential Flow Filtration (TFF) steps: (i) a microfiltration in which the culture supernatant containing the nOMV is separated from the bacteria, and (ii) an ultrafiltration in which the nOMV are separated from soluble proteins (for a general reference see *e.g.* PLoS One. 2015; 10(8): e0134478). The thus obtained nOMV can then directly be used within the present invention without additional purification/isolation steps. The presently considered nOMVs have a preferred size distribution comprised from 20 to 250 nm, measured by Dynamic Light Scattering technique.

According to some embodiments, the nOMVs are prepared from wild-type bacteria or from bacteria which have been genetically manipulated generally to increase immunogenicity (*e.g.* to hyper-express immunogens), to reduce toxicity, to inhibit capsular saccharide synthesis, to down-regulate immunodominant antigen expression, and the like. They also may be prepared from hyperblebbing strains. The nOMVs of the invention may also express exogenous proteins on their surface and they may be endotoxin-depleted.

Preferably, the nOMVs to be used in the present invention are produced from genetically-modified bacterial strains that are mutated to enhance vesicles production, and optionally also to remove or modify antigens (*e.g.* lipid A) and/or to over-express homologous antigens or antigens from other organisms. Said preferred nOMV are also known as Generalized Modules of Membrane Antigens (GMMA) as *e.g.* described in PLoS One. 2015; 10(8): e0134478.

Enhanced spontaneous generation of vesicles can be achieved, for example, by targeted deletion of proteins involved in maintenance of the membrane integrity. It has been observed that the outer surface of nOMVs substantially corresponds to the outer surface of the bacterium from which they are derived, preserving the membrane antigens (including *e.g.* lipopolysaccharides, lipooligosaccharides and lipoproteins) in the context of the membrane. Advantageously, the nOMVs used in the invention (unlike detergent-extracted dOMVs) retain these outer membrane components in their native conformation and correct orientation, better preserving immunogenicity against the bacterial strain from which they are derived.

Generally, the nOMVs for use in the present invention may be prepared from any suitable bacterium, where preferred bacteria include, but are not limited to: *Neisseria (e.g.* in particular *N. meningitidis* of any serogroup including A, B, C, Y, X or W135, or from a non-pathogenic *Neisseria*), *Shigella* (such as *S. sonnei, S. flexneri, dysenteriae or boydii*), *Salmonella enterica serovars* (such as *Salmonella* Paratyphi, *Salmonella* Enteritidis, *Salmonella* Typhi or *Salmonella* Typhimurium), *Haemophilus influenzae* (*e.g.* non-typable *H. influenzae*), *Vibrio cholerae, Bordetella pertussis, Mycobacterium smegmatis, Mycobacterium bovis BCG, Escherichia coli, Bacteroides* (including *Porphyromonas), Pseudomonas aeruginosa, Helicobacter pylori, Brucella melitensis Campylobacter jejuni, Actinobacillus actinomycetemcomitans, Xenorhabdus nematophilus, Moraxella catarrhalis,* or *Borrelia burgdorferi.*

Particularly preferred bacteria are selected from at least one of: *S. sonnei, S. flexneri, Salmonella* bacterium, and *meningococcus.*

Virulent *Shigella* strains possess a 220kb plasmid that mediates virulence properties. This "virulence plasmid" has been shown to encode the genes for several aspects of *Shigella* virulence, including adhesins for target epithelial cells, the invasion plasmid antigens, virF, virG, and the like. A *Shigella* used with the invention may or may not possess a virulence plasmid. Absence of the plasmid can stabilise the strain during industrial culture, attenuate the strain by removing virulence factors (thereby increasing safety of manufacture), avoid the presence of the ShET-2 enterotoxin (encoded by the ospD3 or sen gene on the plasmid), and avoid the presence of msbB2 which is a second copy of the msbB gene responsible for acylation of lipid A.

As far as *Salmonella* bacterium is concerned, a particularly preferred strain is selected from: *Salmonella* Typhimurium, *Salmonella* Enteritidis and *Salmonella* Paratyphi A.

*Meningococcus* bacteria nOMVs are also preferred. Such vesicles can be prepared from any *meningococcal* strain. The vesicles are preferably prepared from a serogroup B strain, but it is also preferred to prepare them from serogroups other than B, such as one of: A, C, W135, Y or X. The strain may be of any serotype (*e.g.* 1, 2a, 2b, 4, 14, 15, 16, etc.), any serosubtype (*e.g.* P1.4), and any immunotype (*e.g.* L1; L2; L3; L3,7; L3,7,9; L10; etc.). The *meningococci* may be from any suitable lineage, including hyperinvasive and hypervirulent lineages, preferably any of the following seven hypervirulent lineages: subgroup I; subgroup III; subgroup IV-1; ET-5 complex; ET-37 complex; A4 cluster; lineage 3. Most preferably, nOMVs are prepared from the strain NZ98/254, or another strain with the P1.4 PorA serosubtype.

In another embodiment, bacteria for preparing nOMVs useful for the invention may be mutant strains which have been manipulated *e.g.* to enhance vesicles production, to express one or more desired antigen(s), and/or to knockout or modify an undesired gene (*e.g.* one which encodes a toxin or which encodes an enzyme involved in generating a toxic product, such as endotoxin).

In this direction, other preferred nOMVs for the invention are produced by a *Salmonella* bacterium, particularly a *S.* Typhimurium (also known as *Salmonella enterica serovar* Typhimurium) which does not express a functional TolR protein.

Where the vesicles are prepared from *E.coli, Shigella* or *Salmonella* the bacterium may express no more than 4 of TolA, TolB, TolQ, TolR and Pal proteins. Thus at least one protein from the natural five-protein Tol-Pal system may be absent, resulting in a bacterium which, during growth in culture medium, releases greater quantities of outer membrane vesicles into the medium than the same bacterium expressing all 5 Tol-Pal proteins. Preferably TolR is not expressed, but the other four proteins may be expressed (*i.e.* a ΔTolR strain).

In preferred embodiments, at least one of the five Tol-Pal proteins in *E.coli, Shigella* or *Salmonella* is removed *e.g.* by deletion or inactivation of the gene encoding the protein. Thus the bacterium may express 0, 1, 2, 3 or 4 of TolA, TolB, TolQ, TolR and Pal proteins. Removal of one of the five proteins can suffice, in which case the bacterium expresses only 4 of these proteins. Preferably the TolR protein is removed *e.g.* by inactivation of a starting strain's tolR gene. Thus a preferred bacterium may be tolA+ tolB+ tolQ+ TolR- Pal+.

In some embodiments, the bacterium expresses all five Tol-Pal proteins, but at least one is mutated to cause hyperblebbing. For instance, the TolA, TolQ, TolR and/or Pal protein may be mutated such that the protein retains its membrane localisation but its interactions with other members of the Tol-Pal system are disrupted. The bacterium will thus retain TolA, TolQ and TolR as transmembrane proteins in the inner membrane, and Pal protein as a periplasm-facing lipoprotein in the outer membrane, but at least one of the TolA, TolQ, TolR and/or Pal proteins is mutated and not fully functional.

In addition, other mutations may also be present *e.g.* to give OAg-deficient strains, such as ΔgalU, ΔgalE or ΔwbaP in *E.coli, Shigella* or *Salmonella* strains.

In one further preferred embodiment, a *meningococcus* does not express a functional MltA protein. Knockout of MltA (the membrane-bound lytic transglycosylase, also known as GNA33) in *meningococcus* provides bacteria which spontaneously release large amounts of nOMVs into culture medium, from which they can be readily purified. For instance, the vesicles can be purified using the two stage size filtration process, comprising: (i) a first filtration step in which vesicles are separated from the bacteria based on their different sizes, with the vesicles passing into the filtrate; and (ii) a second filtration step in which the vesicles are retained in the retentate.

In the present invention, it is preferred that -OAg is present on the nOMVs because it has been observed (*e.g.* nOMVs from *Salmonella* and *Shigella*) that, the presence of the -OAg on the surface of said nOMVs is advantageous in providing a bivalent vaccine, as the -OAg can act as a protective antigen. Some preferred strains have penta- or tetra-acylated less toxic LPS, which includes attached -OAg, after the mutation of msbB, htrB, ddg and/or PagP (see e.g. Rossi O et al, Clin Vaccine Immunol. 2016 Apr 4;23(4):304-14 and Rossi O. et al, J. Biol. Chem. 2014 Sep 5;289(36):24922-35).

In *Neisseria,* the strain has preferably a modified fur gene. According to this embodiment, mutant *Neisseria* are engineered to reduce or switch off expression of at least one gene involved in rendering toxic the lipid A portion of LPS, in particular of lpxl1 gene. In this way, the resulting nOMVs present a reduced toxicity respect to the wild type strain, since the conversion of acylated lipid A in a less acylated form.

Similarly, preferred mutant *Neisseria* for the invention are engineered to reduce or switch off expression of at least one gene involved in the capsular saccharide synthesis or export, in particular of synX and/or ctrA genes. In this way, the resulting nOMVs may present a cross protection versus different serotypes, particularly appreciated by the skilled in the art.

In preferred embodiments a strain may include one or more of the knockout and/or hyper-expression mutations disclosed *e.g.* in Fukusawa et al. (1999), Vaccine 17:2951-2958. For instance, following the therein guidance and nomenclature, useful genes for down-regulation and/or knockout include: (a) Cps, CtrA, CtrB, CtrC, CtrD, FrpB, GalE, HtrB/MsbB, LbpA, LbpB, LpxK, Opa, Opc, PilC, PorB, SiaA, SiaB, SiaC, SiaD, TbpA, and/or TbpB; (b) CtrA, CtrB, CtrC, CtrD, FrpB, GalE, HtrB/MsbB, LbpA, LbpB, LpxK, Opa, Opc, PhoP, PilC, PmrE, PmrF, SiaA, SiaB, SiaC, SiaD, TbpA, and/or TbpB; (c) ExbB, ExbD, rmpM, CtrA, CtrB, CtrD, GalE, LbpA, LpbB, Opa, Opc, PilC, PorB, SiaA, SiaB, SiaC, SiaD, TbpA, and/or TbpB; or (d) CtrA, CtrB, CtrD, FrpB, OpA, OpC, PilC, PorB, SiaD, SynA, SynB, SynX and/or SynC.

As above mentioned, the nOMVs are covalently linked to the bifunctional linker by way of at least one protein residue, generally located on the surface of the vesicle. In this direction, the proteins will preferably react with a terminal end of the linker by means of one or more amino, thiol or hydroxyl amino acid functionalities, being this latter an alpha hydroxyl group or part of the carboxy aminoacid functionality. Preferably, the protein functional group is an amino group, more preferably a primary amine (-NH₂). These functional groups may naturally be present in the amino acid portions of interest, or even introduced artificially for the purposes of conjugation.

When the selected linker is a homobifunctional bivalent linker, it will be understood that the protein functional group and the antigen functional group that will respectively react with the terminal portions of the linker will be preferably the same. By way of example, a lysine aminoacid residue of one or more nOMV proteins will react with the linker (*e.g.* BS3) via the corresponding -NH₂ functional group. In the same way, also a selected antigen (*e.g.* Pfs25) will react with the remaining free terminal portion of the linker via the relevant amino (-NH₂) groups. Of note, and as well explained in the present description, the reaction occurs without substantial cross reaction or aggregation formation, thus leading to the final product, useful *e.g.* for the preparation of multivalent vaccines, in a very reliable and versatile way, and differently from using dOMVs, as indicated in the comparative Example 8 and 8a.

Preferred amino acid residues include, but are not limited to: arginine, lysine, asparagine, glutamine, aspartic or glutamic acid, cysteine and histidine. Preferably, the nOMV proteins are those having one or more aminoacid moiety showing free amino groups, preferably-NH₂ groups. Even more preferably said aminoacid moiety is the arginine and/or lysine, whereby different -NH₂ groups form different arginine and/or lysine proteins are able to selectively react with the linker according to the present invention.

As far as the divalent linker is concerned, this is typically a molecule of a certain length, with a suitable water solubility and polarity, able to covalently bind the nOMV proteins and the antigen by its terminal ends respectively. In order to optimize the solubility of the chosen linker, it may be expedient to introduce one or more polar group such as sulfate, sulfite, phosphate and the like, or even use the corresponding salt thereof, *e.g.* as alkaline or alkaline earth metal salt, where possible. Due to the versatility of the present invention, it is possible to use different linkers, in terms *e.g.* of lengths, polarity, and steric hindrance, thus providing a covalent bond with both the nOMV protein residues and the selected antigen. By that, the invention allows the preparation of a series of final nOMV-linker-antigen conjugates endowed with remarkable and specific behavior, with particular regard to their immunogenicity and activity.

The linker can be heterobifunctional (*i.e.* bearing two different terminal functionalities) or, preferably homobifunctional (*i.e.* having equal terminal functionalities). Even more preferably, the liker is symmetric with respect to a hypothetical vertical axis.

Thus the divalent linker according to the present invention has a general formula **(I):**

X-L-X' (I)

wherein:
- X and X': are different to each other or the same, and are a functional group able to selectively react with nOMV proteins on one hand and with the selected antigen on the other hand, preferably by forming ester, amido or thioester moieties;
- -L-: is a divalent linear or branched C₁-C₁₅ alkyl or alkenyl group optionally substituted, and optionally interrupted by one or more heteroatom selected from: oxygen (-O-), sulfur (-S-), nitrogen (-NH- or optionally substituted -N- group) and the like.

In one embodiment, -L- is preferably a divalent linear C₃-C₁₂ alkyl group, optionally substituted or interrupted by one or more oxygen (-O-) heteroatom. In a still more preferred embodiment, -L- is a divalent linear C₃-C₆ alkyl group.

According to formula (I), the linker is further characterized by having both terminal portions bearing two functionalities X and X' which are preferably the same thus providing a divalent homo-functional linker. In one embodiment, the X and/or X' groups can be any which form esters, thioester or amide when combined with a hydroxyl, thiol or amino functionality respectively.

Preferably, X and/or X' are N-hydroxysuccinimide ester conjugates, more preferably selected from at least one of: 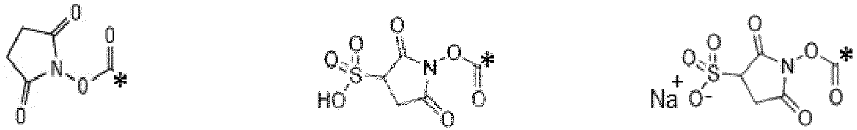 wherein the * represents the point of contact with the -L- spacer in formula (I), as above defined.

Thus, in a still preferred embodiment, the linker is selected from at least one of: disuccinimidyl glutarate (DSG), disuccinimidyl suberate (DSS), succinimidyl 3-(2-pyridyldithio)propionate (SPDP), Succinimidyl 6-(3-[2-pyridyldithio]-propionamido)hexanoate (LC-SPDP), sulfosuccinimidyl 6-(3'-(2-pyridyldithio)propionamido)hexanoate (sulfo-LC-SPDP), 4-succinimidyloxycarbonyl-α-methyl-α-(2-pyridyldithio)toluene (SMPT), sulfosuccinimidyl-6-[α-methyl-α-(2-pyridyldithio)tolueamideo]hexanoate (sulfo-LC-SMPT), succinimidyl 4-(N-maleimidomethyl)cyclohexane-1-carboxylate (SMCC), sulfosuccinimidyl 4-(N-maleimidomethyl)cyclohexane-1-carboxylate (suflo-SMCC), m-maleimidobenzoyl-N-hydroxysuccinimide ester (MBS), m-maleimidobenzoyl-N-hydroxysulfosuccinimide ester (sulfo-MBS), N-succinimidyl (4-iodoacetyl)aminobenzoate (SIAB), sulfosuccinimidyl (4-iodoacetyl)aminobenzoate (sulfo-SIAB), succinimidyl 4-(N-maleimidophenyl)butyrate (SMPB), sulfosuccinimidyl 4-(N-maleimidophenyl)butyrate(sulfo-SMPB), N-γ-maleimidobutyryl-oxysuccinimide ester (GMBS), N-γ-maleimidobutyryl-oxysulfosuccinimide ester (sulfo-GMBS), succinimidyl-6-((((4-(iodoacetyl)amino)methyl)cyclohexane-1-carbonyl)amino)hexanoate (SIACX), succinimidyl 6[6-(((iodoacetyl)amino)hexanoyl)amino]hexanoate (SIAXX), succinimidyl-4-(((iodoacetyl)amino)methyl)cyclohexane-1-carboxylate (SIAC), succinimidyl 6-[(iodoacetyl)amino]hexanoate (SIAX) and p-nitrophenyl iodoacetate (NPIA), N-hydroxysuccinimide, N oxysuccinimide and adipic acid N-hydroxysuccinimide diester (SIDEA) and Bis(sulfosuccinimidyl) suberate (BS3, CAS No. 82436-77-9).

In one embodiment, additional preferred bifunctional linkers reactive with amines for use with the invention are selected from at least one of: acryloyl halides (*e.g.* chloride), ethylene glycol bis[succinimidylsuccinate], bis(sulfosuccinimidyl)tri(ethylene glycol) (BS(PEG)3), bis(sulfosuccinimidyl)tetra(ethylene glycol) (BS(PEG)4), bis(sulfosuccinimidyl)penta(ethylene glycol) (BS(PEG)5) and bis(sulfosuccinimidyl)exa(ethylene glycol) (BS(PEG)6), where bis(sulfosuccinimidyl)penta(ethylene glycol) (BS(PEG)5, CAS No 756526-03-1) is particularly preferred.

Preferred homobifunctional linkers able to react with thiol functional groups on nOMV protein and antigen according to the invention, are those having X and X' selected from at least one of: 2-pyridyldithio, maleimide or iodoacetyl residue.

Other linkers suitable for the reaction with the nOMV protein hydroxyl group as above defined, are selected from at least one of: Adipic acid dihydrazide (ADH), β-propionamido, nitrophenyl-ethylamine, haloacyl halides, 6-aminocaproic acid.

Preferred linkers are selected from at least one of: (BS(PEG)5), Disuccinimidyl glutarate (DSG) or a salt thereof, SIDEA and BS3, where BS3 is even more preferred (for a general reference on BS3 see e.g. US patent 4,965,338). According to a still preferred embodiment, DSG is particularly useful when operating at pH of about 9. Surprisingly, the efficacy of the conjugation reaction can be conveniently increased when (BS(PEG)5) or BS3 are used as divalent linker, substantially in the absence of vesicle aggregates formation. In this respect, it has to be highlighted that the use of BS3 according to the present invention does not provide substantial crosslinking of nOMV surface proteins to form high-molecular-mass aggregates, but rather, selective reaction with nOMV on one terminal end, and with the selected antigen on the other end. This behavior is further supported by the herein enclosed experimental part, where example 8 and example 8a (comparative, using dOMV) are described.

Advantageously, any proper antigen may be conjugated to the nOMV to obtain the nOMV-linker-antigen conjugates of the invention. In any case, the connection of one or more selected antigen produces an immunogenic conjugate which can raise an immune response which recognises said antigen, and which also recognises one or more components in the nOMV, thereby conveniently providing a multivalent vaccine. Antigens will be included in the present conjugates at a concentration which is high enough to elicit, when administered to a host, an immune response which recognises that antigen. Moreover, the immune response is preferably protective against the pathogen from which the antigen was derived, even more preferably against one of the pathogens listed below.

In one embodiment of the invention, the nOMV is conjugated to at least one homologous antigen, *i.e.* derived from the same organism from which the nOMV is derived. In a still preferred embodiment, the selected antigen is a heterologous antigen *i.e.* derived from a different organism from the organism from which the nOMV is derived.

In any case, the antigens may generally be selected from any immunogenic polypeptides, *i.e.* polypeptides able to elicit an immune response when administered to a subject. Polypeptides used with the invention will include an amino acid having a residue, or a side chain, with a functional group suitable for conjugation, preferably an amino or a thiol group, even more preferably of general formula: -NH₂ or -SH. These residues may naturally be present in an antigen, or they may be introduced artificially for the purposes of conjugation. Preferred amino acid residues include, but are not limited to: arginine, lysine, asparagine, glutamine, cysteine and histidine. The most preferred amino acid residue for conjugation is lysine.

Polypeptide antigens conjugated to nOMVs are preferably prepared in substantially pure or substantially isolated form (*i.e.* substantially free from other polypeptides). They can be prepared by various means *e.g.* by chemical synthesis (at least in part), by digesting longer polypeptides using proteases, by translation from RNA, by purification from cell culture (*e.g.* from recombinant expression or from native culture), and the like. Recombinant expression in an *E.coli* host is a useful expression route. Polypeptide antigens can take various forms (*e.g.* native, fusions, glycosylated, non-glycosylated, lipidated, disulfide bridges and the like).

Polypeptide antigens used with the invention have a preferred average molecular weight of at least 1 kDa, more preferably of at least 3.5 kDa, even more preferably from 10 to 80 kDa. Still more preferably, the average molecular weight is comprised from 15 to 75 kDa.

Further preferred polypeptide antigens for conjugating to nOMVs according to the present invention comprise an epitope from a fungal, bacterial, protozoan or viral polypeptide. Preferred protozoan polypeptides are from a *Plasmodium* (such as *P. falciparum, P. vivax, P. ovale*). Particularly preferred bacterial polypeptides are selected from: *E. coli, N. meningitidis,* and *Streptococci* (such as *S.agalactiae, S.pneumoniae, S.pyogenes*).

Preferred *E.coli* polypeptide antigens include CTF1232 (SEQ ID NO: 14), 3526 (SsIE, SEQ ID NO: 15) and 405 (FdeC, SEQ ID NO: 16). As a non-limiting preferred example, nOMV from *Shigella* or *Salmonella* can be conjugated to CTF1232, 3526 or 405, according to the present invention, to generate a bivalent vaccine covering both enterotoxigenic *E. coli* (ETEC) and *Shigella*/*Salmonella.*

In one embodiment, the considered *N.meningitidis* polypeptides are able, when administered to a mammal, to elicit an antibody response that is bactericidal against *meningococcus.* Preferred *N.meningitidis* polypeptides for use with the invention are selected from at least one of: NHBA, NadA, NsPA, NhhA, App and fHbp, as herein below detailed.

### NHBA antigen.

The NHBA antigen was included in the published genome sequence for meningococcal serogroup B strain MC58 as gene NMB2132 (GenBank accession number GI:7227388; SEQ ID NO: 2 herein). The sequences of NHBA antigen from many strains have been published since then. Various immunogenic fragments of the NHBA antigen have also been reported. Preferred NHBA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, e.g. 100%) to SEQ ID NO: 2; and/or (b) comprising a fragment of at least 'n' consecutive amino acids of SEQ ID NO: 2, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 2. The most useful NHBA antigens of the invention can elicit antibodies which, after administration to a subject, can bind to a meningococcal polypeptide consisting of amino acid sequence SEQ ID NO: 2. Advantageous NHBA antigens for use with the invention can elicit bactericidal *anti-meningococcal* antibodies after administration to a subject.

### NadA antigen.

The NadA antigen was included in the published genome sequence for *meningococcal* serogroup B strain MC58 (see *e.g.* Tettelin et al. (2000) Science 287:1809-1815) as gene NMB1994 (GenBank accession number GI:7227256; SEQ ID NO: 3 herein). The sequences of NadA antigen from many strains have been published since then, and the protein's activity as a *Neisserial* adhesin has been well documented. Various immunogenic fragments of NadA have also been reported. Preferred NadA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, *e.g.* 100%) to SEQ ID NO: 3; and/or (b) comprising a fragment of at least 'n' consecutive amino acids of SEQ ID NO: 3, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 3. The most preferred NadA antigens of the invention can elicit antibodies which, after administration to a subject, can bind to a *meningococcal* polypeptide consisting of amino acid sequence SEQ ID NO: 3. Advantageous NadA antigens for use with the invention can elicit bactericidal anti-*meningococcal* antibodies after administration to a subject. SEQ ID NO: 7 is one such fragment.

### NspA antigen.

The NspA antigen was included in the published genome sequence for *meningococcal* serogroup B strain MC58 (see *e.g.* Tettelin et al. (2000) Science 287:1809-1815) as gene NMB0663 (GenBank accession number GI:7225888; SEQ ID NO: 4 herein). The sequences of NspA antigen from many strains have been published since then. Various immunogenic fragments of NspA have also been reported. Preferred NspA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, *e.g.* 100%) to SEQ ID NO: 4; and/or (b) comprising a fragment of at least 'n' consecutive amino acids of SEQ ID NO: 4, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 4. The most preferred NspA antigens of the invention can elicit antibodies which, after administration to a subject, can bind to a meningococcal polypeptide consisting of amino acid sequence SEQ ID NO: 4. Advantageous NspA antigens for use with the invention can elicit bactericidal anti-*meningococcal* antibodies after administration to a subject.

### NhhA antigen.

The NhhA antigen was included in the published genome sequence for *meningococcal* serogroup B strain MC58 (see *e.g.* Tettelin et al. (2000) Science 287:1809-1815) as gene NMB0992 (GenBank accession number GI:7226232; SEQ ID NO: 5 herein). The sequences of NhhA antigen from many strains have been published since *e.g.* WO00/66741 and WO01/55182, and various immunogenic fragments of NhhA have been reported. It is also known as Hsf. Preferred NhhA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, e.g. 100%) to SEQ ID NO: 5; and/or (b) comprising a fragment of at least 'n' consecutive amino acids of SEQ ID NO: 5, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 5. The most preferred NhhA antigens of the invention can elicit antibodies which, after administration to a subject, can bind to a *meningococcal* polypeptide consisting of amino acid sequence SEQ ID NO: 5. Advantageous NhhA antigens for use with the invention can elicit bactericidal *anti-meningococcal* antibodies after administration to a subject.

### App antigen.

The App antigen was included in the published genome sequence for *meningococcal* serogroup B strain MC58 (see *e.g.* Tettelin et al. (2000) Science 287:1809-1815) as gene NMB1985 (GenBank accession number GI:7227246; SEQ ID NO: 6 herein). The sequences of App antigen from many strains have been published since then. Various immunogenic fragments of App have also been reported. Preferred App antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, *e.g.* 100%) to SEQ ID NO: 6; and/or (b) comprising a fragment of at least 'n' consecutive amino acids of SEQ ID NO: 6, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 6. The most preferred App antigens of the invention can elicit antibodies which, after administration to a subject, can bind to a *meningococcal* polypeptide consisting of amino acid sequence SEQ ID NO: 6. Advantageous App antigens for use with the invention can elicit bactericidal *anti-meningococcal* antibodies after administration to a subject.

### fHbp antigen.

The factor H binding protein exists as three variants (v1, v2 and v3), and the invention can use any of these as preferred embodiment.

A v1 fHbp preferably comprises (a) an amino acid sequence which has at least k'% identity to SEQ ID NO: 8, and/or (b) a fragment of SEQ ID NO: 8. k' refers to percentage identity and could be defined as any number from 1 to 100. With reference to amino acid or nucleic acid sequences, generally the identity used in the application starts from as low as 40% with specific references to higher percentages, *e.g.* 60%, 65%, 70%, 75%, 80%, 85%, 90%, 91%, 92%, 93%, 94%, 95%, 96%, 97%, 98%, 99%, 99.5% or more, e.g. 100%.

The fragment will preferably include at least one epitope from SEQ ID NO: 8. Preferably, the v1 fHbp can elicit antibodies which are bactericidal against v1 strains *e.g.* against strain MC58 (available from the ATCC as 'BAA-335').

A v2 fHbp preferably comprises (a) an amino acid sequence which has at least k'% identity to SEQ ID NO: 1, and/or (b) a fragment of SEQ ID NO: 1. Information about 'k' and fragments are given above. The fragment will preferably include at least one epitope from SEQ ID NO: 1. Preferably, the v2 fHbp can elicit antibodies which are bactericidal against v2 strains e.g. against strain M2091 (ATCC 13091).

A v3 fHbp preferably comprise (a) an amino acid sequence which has at least k'% identity to SEQ ID NO: 9, and/or (b) a fragment of SEQ ID NO: 9. Information about 'k' and fragments are given above. The fragment will preferably include at least one epitope from SEQ ID NO: 9. Preferably, the v3 fHbp can elicit antibodies which are bactericidal against v3 strains *e.g.* against strain M01-240355.

Antigens from Group A *Streptococcus* (GAS), Group B *Streptococcus* (GBS) and *Pneumococcus* are also equally preferred. As non-limiting examples, GAS25 (Slo), GAS40 (SpyAD) and GAS57 (SpyCEP) antigens can be incorporated into conjugates in accordance with some embodiments of the invention.

Plasmodium antigens are further preferred. These can be from any suitable species, where preferred species are selected from: *P.falciparum, P.vivax* and *P.ovale.*

Still another preferred antigen is Pfs25 (SEQ ID NO: 10), which is a sexual stage antigen of *P.falciparum* expressed on the surface of zygote and ookinete forms of the parasite. Another preferred antigen is Pfs48/45, which is a transmission-blocking vaccine candidate. Recently the C-terminal 10 cysteine fragment (10C) of Pfs48/45, containing three known epitopes for transmission blocking antibodies, has been produced as a chimera with the N-terminal portion of GLURP (RO), the asexual blood-stage antigen glutamate-rich protein. The resulting fusion protein (RO10C) elicited high levels of transmission-blocking antibodies in rodents (see Theisen et al. (2014) Vaccine 32:2623-2630). Shing et al. (2015) Vaccine 33:1981-1986 describes a chimera containing truncated 6C-fragments, which increases the yield of correctly-folded conformer. The RO6C construct was able to elicit high titer transmission blocking antibodies in rats. RO6C (SEQ ID NO: 11) is a preferred antigen that can be conjugated according to the present invention.

Another preferred antigen is the *circumsporozoite* protein (CSP; SEQ ID NO: 12).

Shorter peptides from CSP may also be conjugated according to the present invention. For example, the 12 amino acid (NANP)₃ peptide (SEQ ID NO: 13) derived from CSP can be used according to preferred embodiments.

In another still preferred embodiment the antigens are a saccharide species. The invention is in fact also suitable for conjugating one or more selected saccharide antigens to nOMVs, whereby saccharides may be used in their full-length natural form. As an alternative, a particular size fraction can also advantageously be selected. Thus, saccharides may be fragmented from their natural length, and optionally a size fraction of these fragments can be used. Even further, the saccharides are not limited to saccharides purified from natural sources and synthetic or semi-synthetic saccharides can be used instead. The polysaccharide antigen to be used according to the invention is generally functionalized in order to allow the reaction with the divalent linker. Thus, in one embodiment, when the antigen is a polysaccharide, the invention comprises the oxidation of the anomeric hydroxyl group of said antigen to aldehyde, followed by reductive amination conversion to amino group, e.g. using NalO₄ and NaBH₄, according to procedures known in the art. In this way, the polysaccharide antigen will present a suitable -NH₂ group for the reaction with the terminal portion of the linker, already connected to the nOMV to give nOMV-Linker-antigen conjugate.

Preferred saccharide antigens are bacterial capsular saccharides (CPSs). These include, but are not limited to, the capsular saccharides selected from at least one of: *Haemophilus influenzae* type B; *Neisseria meningitidis* serogroups A, C, W135, X and Y, where serogroups A and C are particularly preferred; *Streptococcus pneumoniae* serotypes 1, 2, 3, 4, 5, 6A, 6B, 7F, 8, 9N, 9V, 10A, 11A, 12F, 14, 15B, 17F, 18C, 19A, 19F, 20, 22F, 23F, and 33F; *Salmonella* including *Salmonella enterica serovar* Typhi Vi, either full length or fragmented (indicated as fVi); *Streptococcus agalactiae* serotypes Ia, Ib, and III; Streptococcus pyogenes, *Shigella* sp.

In a preferred embodiment, the nOMV is a GMMA from *Meningococcus* bacteria, preferably prepared from a serogroup B strain, and the selected antigen is a capsular saccharide from *Neisseria meningitidis* serogroups A or C, even more preferably conjugated to the GMMA via a SIDEA linker.

In any case, and as above mentioned, the selected antigens could be conjugated to nOMVs derived from the same or even from a different bacterial strain, thus providing a multivalent vaccine. In this respect, in a more preferred embodiment of the invention, the nOMV and the saccharide antigen are derived from different bacterial strains.

Other preferred saccharide antigens are β-glucans, particularly useful for protecting against *C.albicans* (for a general reference see Sandlin et al. (1995) Infect. Immun., 63:229-37).

Other preferred saccharide antigens are poly-rhamnose oligosaccharides for protecting against Group A *Streptococcus* (GAS). Native GAS saccharide has a poly-rhamnose backbone substituted with NAcGlcN. Synthetic oligosaccharides of poly-rhamnose, or oligomers with the structure of native GAS saccharide, can be conjugated to nOMVs according to the invention.

The conjugates of the invention are immunogenic, as demonstrated by the studies in mice and supported by the herein included experimental part.

As above set forth, in a further aspect, the invention refers to a process for preparing nOMV-linker-antigen conjugates, comprising the reaction of at least a nOMV protein residue with a first terminal portion of a divalent linker, followed by reaction of the second terminal portion of such linker with a selected foreign antigen, as herein below described in more details.

Thus, according to an embodiment, the invention refers to a process for the preparation of nOMV-linker-antigen conjugates, comprising the step of:
(i) reacting at least one protein on the nOMV surface with a divalent linker to form a nOMV-linker intermediate; and
(ii) reacting the thus obtained nOMV-linker intermediate with at least a foreign antigen to form the nOMV-linker-antigen conjugates of the invention.

According to an embodiment, the nOMVs are initially suspended in a proper buffer, such as MES (2-(N-morpholino)ethanesulfonic acid) buffer, borate buffer or PBS (phosphate buffer saline), in order to select a pH comprised from 5 to 9, preferably from 5 to 7 or from 7 to 9, depending for instance on the selected linker or conjugation conditions. Same pH ranges are also used for the step ii) for the reaction between the free terminal end of the linker and the selected antigen as herein below described in details.

After step i) the percentage of nOMV functionalization is comprised from 15% to 60%, mainly depending on the kind of divalent linker used. To his regards, the % of reactive functional groups at the free terminal end of the linker is from 15% to 40%, preferably from 30% to 35%, depending on the kind and stability of said reactive functional groups. It is in fact noticed that such ranges allow for an implemented efficacy of the process, thus resulting in higher amount of final antigen conjugate of the invention.

The thus obtained buffered suspension has a nOMV concentration comprised from 2 and 10 mg/mL, preferably from 3 to 6 mg/mL. The chosen linker, is generally added in amounts depending e.g. on the -NH₂ groups on the nOMV, preferably in excess, even more preferably comprised from 10 to 20 equivalents per mole of -NH₂.

Depending on the linker, it could be convenient to preventively solve it in a polar dry solvent, such as DMSO or the like, in order to facilitate the handling and the efficacy, thus obtaining improved results in terms of overall yield and reproducibility.

The mixture is then incubated at room temperature (e.g. from about 15 to 40° C) for a period of time generally from 30 minutes to 4 hours. Subsequently, the thus obtained nOMV-linker intermediate is purified, e.g. by ultracentrifugation, and then reacted with the selected antigen according to step ii). The antigen is generally solved in a proper buffer solution, such as phosphate buffered saline. The antigen is preferably added in an amount ranging from 2:1 to 1:2 w/w ratio over the nOMV-linker intermediate, or more preferably in a 1:1 ratio. The reaction can be monitored *e.g.* by HPLC/SEC and the final product formation can be confirmed by SDS page/western blot analysis.

As an alternative embodiment, the conjugation reaction comprises the steps of: (i) reacting the antigen with a linker to form an antigen-linker intermediate; and (ii) reacting at least one protein on the nOMV with the thus formed antigen-linker intermediate to form a nOMV-linker-antigen conjugate.

As another alternative, the conjugation reaction comprises the steps of: (i) reacting the antigen with a first linker to form an antigen-linker intermediate; (ii) reacting at least one protein on the nOMV with a second linker to form a nOMV-linker intermediate; and (iii) reacting the antigen-linker with the nOMV-linker to form a nOMV-linker-antigen conjugate.

The skilled person will understand that when a homobifunctional linker is used, the protein and the antigen functional groups involved in the reaction will be the same chemical entity, as above explained in details.

Thus, according to one preferred embodiment, the process of the present invention comprises the steps of:
(i) reaction of a -NH₂ group of at least a nOMV protein with a divalent homobifunctional linker to form a nOMV-linker intermediate; and
(ii) reaction of said intermediate with a -NH₂ group on a selected foreign antigen to form nOMV-linker-antigen conjugate of the invention.

This type of conjugation reaction is illustrated in Scheme 2 below, using, by way of example, a GMMA as native vesicle and BS3 as the linker.

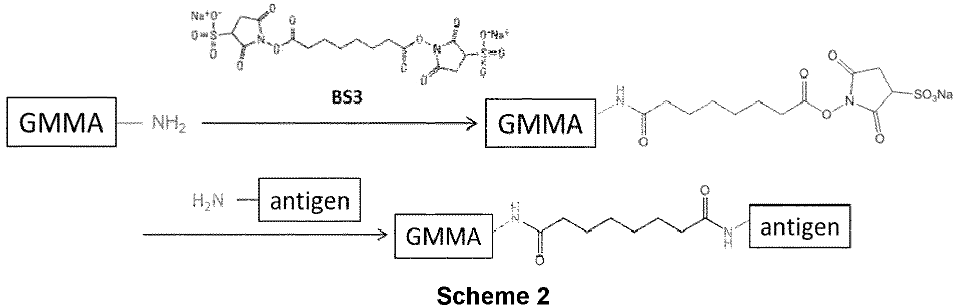

Where the linker reactions with the protein on the surface of the nOMV and the antigen involve different functional groups (such as an amine on the protein on the vesicle and a thiol on the antigen, or vice versa) it is preferred to use a heterobifunctional linker of the above general formula (I) X-L-X', where X and X' are different to each other, as above defined, and L is a moiety as above defined. The X group can react with one functional group, e.g. an amine on the nOMV protein; whereas X' group can react with a different functional group, e.g. a thiol on the selected antigen. Preferably, the X group is N-hydroxysuccinimide or N-oxysuccinimide, whereas the X' group is selected from at least one of: 2-pyridyldithio, maleimide or iodoacetyl group.

An alternative conjugation process of the invention includes reacting the antigen with a first linker and a protein on the vesicle with a second linker, then reacting the first and second linker together to form the conjugate.

By way of example, either the antigen or the protein on the nOMV may be reacted with a linker terminating in a maleimide group, for instance by reacting a primary amine or either the antigen or the protein on the nOMV with succinimidyl-4-(N-maleimidomethyl)cyclohexane-1-carboxylate (SMCC) or N-(γ-maleimidobutyryloxy)succinimide ester (GMBS). A thiol on either the antigen or the protein on the nOMV may then be reacted with the maleimide. The thiol may be native to the protein on the nOMV or antigen or the thiol may be the result of reacting the protein on the nOMV or antigen with a separate linker. This type of conjugation reaction is exemplified in Scheme 3 below, using, by way of example, GMMA and fHbp as the antigen:

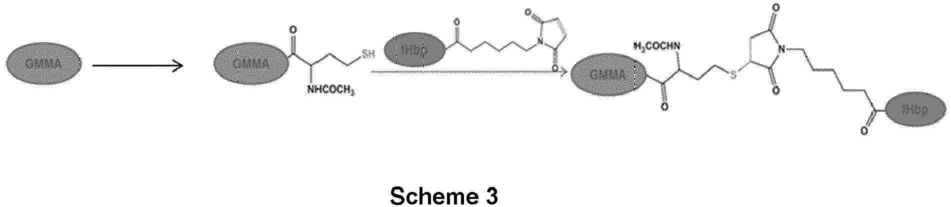

Advantageously, due to its versatility, the invention can be used for the preparation of a variety of conjugates, particularly appreciated by the skilled person when faced with the problem to find convenient and reliable methodologies for obtaining immunogenic conjugates.

As a further alternative, a protein on the vesicle may be linked to the antigen by (i) modifying the vesicle protein to include an alkyne; (ii) modifying the antigen to include an azide, then (iii) reacting the alkyne and azide, known as "click chemistry", as exemplified in Example 6. Alternatively, the antigen may be modified to include an alkyne and the vesicle may be modified to include an azide. This type of conjugation reaction is illustrated in Scheme 4 below, using, by way of example, GMMA as the vesicle and fHbp as the antigen and using copper free click chemistry.

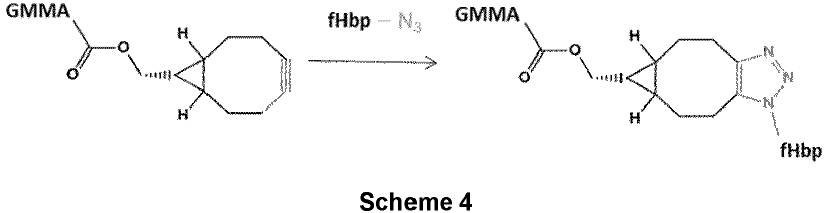

The present invention is also useful for the preparation of variously functionalized nOMVs, allowing multivalent presentation of different antigens on the surface of the selected vesicle.

Thus, according to a preferred embodiment, the invention refers to an immunogenic conjugate comprising a nOMV as above set forth, having at least a protein moiety connected to a first antigen via a divalent linker, wherein said first antigen is connected to a second different antigen. In this direction, the two selected antigens (herein indicated as Ag1 and Ag2) may be coupled together to give an Antigen1-Antigen2 derivative (Ag1-Ag2), which can be subsequently connected to the selected nOMV surface protein moiety by a suitable divalent linker as above described, to give a conjugate indicated by the general formula (I):

nOMV -linker-Ag1-Ag2 (I)

Alternatively, the nOMV protein residue can first be connected to the selected Ag1 via a linker as above described, and subsequently a second Ag2 is further connected to said Ag1 residue, to give the conjugates of the above general formula (I).

In any case, preferred nOMV are the GMMA vesicles. The Ag1 and Ag2 in the formula (I) can be selected among the preferred antigens as above described, either protein or polysaccharide moieties. Preferably, the antigens used for the multi functionalization as herein contemplated are both proteins or both polysaccharides.

According to a further embodiment, the present invention refers to an immunogenic conjugate comprising a nOMV, having at least a surface protein moiety connected to a first antigen (Ag1) by a divalent linker, and at least another surface protein moiety connected to a second different antigen (Ag2) by a divalent linker, to give a conjugate indicated by the general formula (II):

Ag1-linker-nOMV-linker-Ag2 **(II)**

As indicated in formula (II) Ag1 and Ag2 are each individually connected to the nOMV via a suitable linker. In this direction, the linker for the connection of the nOMV protein to the Ag1 or Ag2 can independently be the same or differ from each other.

The conjugates of formula (II) can advantageously provide selective multi functionalization of nOMV, preferably GMMA, by using a specific functionalization pattern. The Ag1 and Ag2 in the formula (II) can be selected among the preferred antigens as above described, either protein or polysaccharide moieties. Preferably, the antigens used for the multi functionalization as herein contemplated are both proteins or both polysaccharides.

Thus according to a more preferred embodiment, the invention refers to a conjugate of formula (II) wherein Ag1 comprises the capsular saccharide of *Nesseria mengitidis* serogroup A (MenA) and Ag2 comprises the capsular saccharide of *Nesseria mengitidis* serogroup C (MenC), even more preferably having the nOMV which is a GMMA vesicle, more preferably obtained from *Nesseria mengitidis* serogroup B, even more preferably expressing fHbp v2 and v3.

Even more preferably, the two linkers, each connecting Ag1 and Ag2, preferably MenA and MenC respectively, are the same, more preferably SIDEA. In this respect, the obtained multifunctionalized GMMA can be used as immunogenic agent against the Neisseria bacteria from MenB, MenA or MenC, as supported by the SBA data collected in the present experimental part.

In one embodiment, said conjugates are prepared by a process comprising adding a mixture of activated MenA and MenC polysaccharides to GMMA, according to the present procedure as above described. The MenA and MenC polysaccharides are preferably activated by reaction with SIDEA, thus providing suitable SIDEA-MenA and SIDEA-MenC derivatives, able to react with the -NH₂ groups of the nOMV protein through the terminal portion of the SIDEA moiety, thus leading to the conjugates of the above general formula (II).

Western blot and HPAEC-PAD analysis confirms the formation of the conjugates where the MenA and the MenC saccharides are randomly connected to the GMMA surface proteins via the linker, and no aggregation of nOMV is detected by dls.

The skilled person will recognize that in light of the versatility of the proposed technology, the present invention may be suitable used for the multi functionalization of nOMV, preferably GMMA, even with more than 2 different antigens. Beside the possibility of selecting different antigens as above indicated, the present invention also allows for the introduction of different amounts of different antigens, thus modulating the antigen/nOMV ratio according e.g. to the selected antigen or used nOMV.

Thus, according to additional embodiments, the invention refers to conjugates of general formula (II) wherein the Ag1 comprises Hib and Ag2 comprises Hia, where said Ag1 and Ag2 are each individually conjugated to a GMMA, preferably a pertussis GMMA, even more preferably by a BS3 linkers. In a still additional embodiments, the invention refers to conjugates of general formula (II) wherein the Ag1 comprises ETEC 405 (FdeC, Seq ID NO:16) and Ag2 comprises ETEC3526 (SsIE, Seq ID NO:15), where said Ag1 and Ag2 are each independently conjugated to a GMMA, preferably a Shigella Sonnei GMMA, even more preferably by a BS3 linker.

According to a further aspect, the invention refers to the conjugates of the invention for use as a medicament, particularly as immunogenic agent, even more preferably for one or more of the pathogens as herein indicated. In other words, the invention refers to the use of the present conjugates for the manufacture of an immunogenic composition.

According to a further aspect, the invention refers to an immunogenic composition, preferably a vaccine, comprising a conjugate of the invention and at least one additional pharmaceutically acceptable carrier, excipient or adjuvant. Generally, pharmaceutically acceptable carrier, excipient or adjuvant can be any substance that does not itself induce the production of antibodies harmful to the patient receiving the composition, and which can be administered. Pharmaceutically acceptable carriers and excipient are those used in the art, and can include liquids such as water, saline, glycerol and ethanol. Auxiliary substances, such as wetting or emulsifying agents, pH buffering substances, and the like, can also be present in such vehicles, according to the prior art.

The invention also provides a method for raising an immune response in a vertebrate, preferably a mammal, comprising administering a conjugate of the invention to the mammal or other vertebrate. The invention also provides conjugates of the invention for use in such methods. The immune response is preferably protective and preferably involves antibodies. The method may raise a booster response.

The mammal is preferably a human. The subject in which disease is prevented may not be the same as the subject that receives the conjugate of the invention. For instance, a conjugate may be administered to a female (before or during pregnancy) in order to protect offspring (so-called maternal immunisation'). Conjugates of the invention may also be used to immunise other mammals *e.g.* cattle, sheep and pigs (especially against *Salmonella* sp.), and other non-mammal vertebrates including fish and poultry.

The invention provides conjugates of the invention for use in therapy (e.g. as immunogenic compositions or as vaccines). The invention also provides a conjugate of the invention for use in a method for raising an immune response in a vertebrate, preferably a mammal. The invention also provides the use of a conjugate of the invention in the manufacture of a medicament for raising an immune response in a vertebrate, preferably a mammal. The uses and methods are particularly useful for preventing/treating a variety of diseases, depending on the antigens and nOMVs within the conjugates as above set forth. Preferred conjugates of the invention can confer an antibody titre in a patient that is superior to the criterion for seroprotection for each antigenic component for an acceptable percentage of human subjects. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

Immunogenic compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration is preferred *e.g.* to the thigh or the upper arm. Injection may be via a needle (*e.g.* a hypodermic needle), but needle-free injection may alternatively be used. The invention may also be used to elicit systemic and/or mucosal immunity. Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g.* between 4-16 weeks), and between priming and boosting, can be routinely determined.

Infections affect various areas of the body and so the compositions of the invention may be prepared in various forms. For example, the compositions may be prepared as injectable, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops. Compositions suitable for parenteral injection are most preferred. The composition is preferably sterile. It is preferably pyrogen-free. It is preferably buffered e.g. at between pH 6 and pH 8, generally around pH 7. Compositions of the invention may be isotonic with respect to humans. Immunogenic compositions comprise an immunologically effective amount of a conjugate of the invention, as well as any other of other specified components, as needed. Dosage treatment may be a single dose schedule or a multiple dose schedule (*e.g.* including booster doses). The composition may be administered in conjunction with other immunoregulatory agents.

Adjuvants which may optionally be used in compositions of the invention include, but are not limited to insoluble metal salts, oil-in-water emulsions (*e.g.* MF59 or AS03, both containing squalene), saponins, non-toxic conjugates of LPS (such as monophosphoryl lipid A or 3-O-deacylated MPL), immunostimulating oligonucleotides, detoxified bacterial ADP-ribosylating toxins, microparticles, liposomes, imidazoquinolones, or mixtures thereof. Other substances that act as immunostimulating agents are disclosed for instance in Watson, Pediatr. Infect. Dis. J. (2000) 19:331-332. The use of an aluminium hydroxide and/or aluminium phosphate adjuvant is particularly preferred. These salts include oxyhydroxides and hydroxyphosphates. The salts can take any suitable form (*e.g.* gel, crystalline, amorphous, etc.).

Conjugates of the invention which include nOMVs from one pathogen and a selected antigen from a second pathogen can be useful as immunogenic compounds for the preparation of multivalent vaccines.

Preferred nOMV-linker-antigen conjugates of the invention are indicated in the following Table 1.

**Table 1: preferred conjugates of the invention**

| ***nOMV*** | ***linker*** | ***Antigen*** |
|---|---|---|
| *Salmonella* Typhimurium | BS3 | Plasmodium falciparum Pfs25 |
| *Salmonella* Typhimurium | BS(PEG)₅ | Plasmodium falciparum Pfs25 |
| *Salmonella* Typhimurium | BS3 | fHbp (*Neisseria meningitidis*) |
| *Salmonella* Typhimurium | BS(PEG)₅ | fHbp (*Neisseria meningitidis*) |
| *Salmonella* Typhimurium | BS3 | Plasmodium falciparum RO6C |
| *Salmonella* Typhimurium | BS(PEG)₅ | Plasmodium falciparum RO6C |
| *Salmonella* Typhimurium | BS3 | Plasmodium falciparum CSP |
| *Salmonella* Typhimurium | BS(PEG)₅ | Plasmodium falciparum CSP |
| *Meningococcal B* | BS3 | fHbp (*Neisseria meningitidis*) |
| *Meningococcal B* | BS(PEG)₅ | fHbp (*Neisseria meningitidis*) |
| *Meningococcal B* | BS3 | *NHBA* (*Neisseria meningitidis*) |
| *Meningococcal B* | BS(PEG)₅ | NHBA (*Neisseria meningitidis*) |
| *Salmonella* Typhimurium | BS3 | Synthetic or native GAS PS |
| *Salmonella* Typhimurium | BS(PEG)₅ | Synthetic or native GAS PS |
| *Salmonella* Typhimurium | BS3 | Synthetic or native GBS PS |
| Meningococcal B | BS3 | Synthetic or native GAS PS |
| Meningococcal B | BS3 | Hib PS |
| *B. Pertussis* | BS3 | Hib PS |
| *Salmonella* Typhimurium | BS3 | Vi PS |
| *Shigella* | BS3 | ETEC 405 |
| *Shigella* | BS3 | ETEC 3526 |
| *Shigella* | BS3 | ETEC CTF1232 |
| Meningococcal B | SIDEA | Capsular saccharide from MenA |
| Meningococcal B | SIDEA | Capsular saccharide from MenC |

Thus, the present conjugates are particularly useful as immunogenic agents against the pathogens listed in Table 1. In particular, data confirmed that GMMA from MenB conjugated to capsular saccharides from MenA and/or MenC show r/hSBA results comparable or even better that those obtained using CRM197 as carrier protein. The SBAs data in fact confirm the immunogenic response against MenA and MenC as well as against MenB (see in particular Examples 12, 13 and 14).

This means that the present conjugates can be suitable used for the preparation of a multivalent immunogenic composition, or a vaccine, for instance, but not only, against MenB and MenC and/or MenA, as herein described and supported in details.

The invention will be now described by the following experimental part, without posing any limitation to its scope.

### EXPERIMENTAL PART

### EXAMPLE 1: nOMV production

The nOMVs as used in the Examples are GMMA vesicles, prepared from S. Typhimurium or N. *meninigitidis* B strains, as *e.g.* disclosed in Clin Vaccine Immunol. 2016 Apr; 23(4): 304-314Characteristics of said nOMVs were as indicated in the following Table 2.

**Table 2: characteristics of purified nOMVs prepared from S. Typhimurium or N. meninigitidis B strains.**

| | ***S. Typhimurium* 1418 (ΔtolR)** | ***S. Typhimurium* 2192 (ΔtolR ΔpagP ΔmsbB)** | ***MenB* (ΔlpxL1, ΔsynX, Δgna33 and ΔfHbp)** |
|---|---|---|---|
| Diameter (nm) by dls | 131.5 | 112.6 | 95.6 |
| nmol Lipid A / mg vesicles by HPLC-SEC/semicarbazide | 172.8 | 243.2 | 68.6 |
| OAg / total protein weight ratio | 0.84 | 0.93 | only LOS present |

### EXAMPLE 2: nOMV conjugation via BS3 linker (Pfs25-S. Typhimurium nOMV conjugation)

Malaria antigen Pfs25 was conjugated to the S. Typhimurium nOMV vesicles following the procedure described below. nOMV, at a protein concentration of 4 mg/mL in 100 mM borate buffer pH 9, were added of BS3 linker (48 mg/mL in the reaction mixture). The mixture was incubated at the controlled temperature of 25°C for 30 minutes. After this time, activated nOMV were purified by ultracentrifuge (110000 rpm, 16 min, 4°C) and immediately added of Pfs25 antigen. In the conjugation step, a 1:1 ratio of nOMV:Pfs25 was used, at a Pfs25 concentration of 2.7 mg/ml in PBS with overnight incubation at room temperature. The conjugate was purified by ultracentrifuge (110000 rpm, 16 min, 4°C) and re-suspended in PBS. Characterization by SDS page/western blot analysis confirmed conjugate formation. Analysis by competitive ELISA showed 19.2% Pfs25 to total protein w/w ratio in the final conjugate. Intermediate activated nOMV were characterised by 62% NH₂ groups derivatised with the linker and 32% active ester groups introduced on NH₂ groups on nOMV. No crosslinking was verified in both the steps of nOMV activation with BS3 and following conjugation, as verified by dls

### EXAMPLE 3: fHbp v2 linked to S. Typhimurium nOMV by BS3 chemistry (recycling fHbp)

fHbp v2 antigen was conjugated to S. Typhimurium nOMV by BS3 chemistry. The reaction was performed by working with fHbp to nOMV-BS3 w/w ratio of 1, fHbp v2 concentration of 0.92 mg/mL in PBS. A further conjugate was produced by recycling unreacted fHbp v2 from the first conjugation batch and re-using it for conjugation with a fresh batch of nOMV-BS3, by using same conjugation condition. SDS PAGE western blot analysis confirmed conjugate formation also with the recycled fHbp v2.

### EXAMPLE 4: CSP and (NANP)₃-SH linked to S. Typhimurium nOMV by SH-malemido chemistry according to the invention

### Example 4.1: CSP derivatization with N-ε-malemidocaproyl-oxysuccinimde ester (EMCS) linker.

CSP at the concentration of 270 µg/mL in PBS was added of EMCS linker (as a 10 mg/mL solution in DMSO) to have a 1:1 molar ratio of linker to Lys residues of the protein. The solution was mixed at RT for 4 hours and then the derivatised protein was purified by PD10 column against MES 10 mM pH 6. The resulting product was characterised by micro BCA (64% recovery).

### Example 4.2: S. Typhimurium nOMV derivatization with SH linker.

nOMV were re-suspended in 100 mM borate buffer pH 8 and added of the activation buffer containing 2.6 mg/mL DTT, 13.16 mg/mL EDTA and 7.04 mg/mL N-acetyl-DL-homocysteine thiolactone linker in 100 mM borate buffer pH 11. nOMV were at 2.3 mg/mL with a molar ratio of linker to NH₂ groups on GMMA equal to 7. nOMV-SH were purified by ultracentrifugation (110000 rpm, 4°C, 1 hour) and recovered in 100 mM MES buffer pH 6. nOMV-SH were characterised by micro BCA (80% protein recovery) and TNBS showing 54% NH₂ groups were activated.

### Example 4.3: S. Typhimurium nOMV derivatization with EMCS linker.

nOMV were resuspended in 100 mM NaPi pH 7.2 and added of EMCS linker (as a 20 mg/mL solution in DMSO) to have a 0.6:1 molar ratio of linker to Lys residues of the protein and with nOMV concentration of 3.94 mg/mL. The reaction was left mixing at RT for 4 hours and nOMV-EMCS were purified by ultracentrifugation (110000 rpm, 4°C, 1 hour). Derivatised nOMV were recovered in 100 mM MES buffer pH 6. nOMV-EMCS were characterised by micro BCA (87% protein recovery) and TNBS and Ellman colorimetric methods for assessing the % of NH₂ groups activated (30%).

### Example 4.4: Conjugation of nOMV-SH with CSP-EMCS.

CSP protein, previously derivatised with EMCS linker, was conjugated to nOMV-SH. Conjugation was performed with 1:1 w/w nOMV to CSP ratio at CSP concentration of 96.7 µg/mL in 100 mM MES pH 6. The reaction was left mixing at RT for 4 hours and the conjugate was purified by ultracentrifuge (110000 rpm, 4°C, 1 hour) and recovered in PBS. Amount of conjugated CSP respect to total protein content was of 0.33 (w/w) as evaluated by HPLC-SEC analysis of the conjugation mixture. SDS PAGE/western blot analysis confirmed conjugate formation.

### Example 4.5: Conjugation of nOMV-EMCS with (NANP)₃-SH.

(NANP)₃-SH protein, having a terminal Cys residue, was added to nOMV-EMCS in MES 100 mM pH 6 at a concentration of 8.7 mg/mL and with a w/w ratio of 1 respect to nOMV. The reaction was left mixing at RT for 4 hours and the conjugate was purified by ultracentrifuge (110000 rpm, 4°C, 1 hour) and recovered in PBS. Amount of conjugated (NANP)₃ respect to total protein content was of 0.31 (w/w) as calculated by Ellman and HPAEC-PAD analysis on the conjugate. SDS PAGE/western blot analysis confirmed conjugate formation.

### EXAMPLE 5: immunogenicity of conjugates of Example 4 in mice

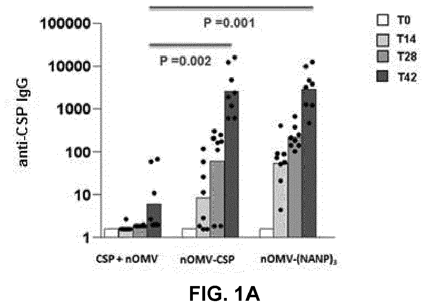

Mice were immunised subcutaneously at days 0 and 28 with nOMV-CSP and nOMV-(NANP)₃ conjugates prepared according to Example 4. Compared to the physical mixture nOMV + CSP, at 2 µg CSP or (NANP)₃ per dose. Anti-CSP IgG titers were measured at days 0, 14, 28 and 42, and results are shown in Figure 1A.

Both nOMV conjugates induced higher anti-CSP specific response than CSP physically mixed with nOMV (Kruskal-Wallis test with Dunn's post hoc analysis, p = 0.002 for nOMV-CSP and p = 0.001 for nOMV-(NANP)₃ respectively at day 42).

Both the conjugates showed the ability to boost the response (day 14-day 42).

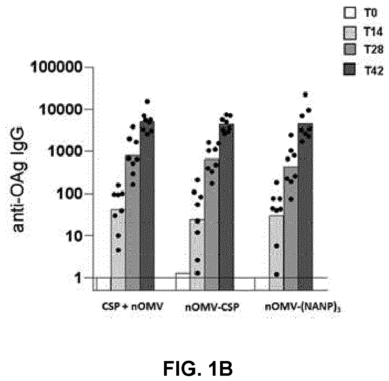

IgG titers against the -OAg were also assessed. The presence of CSP or (NANP)₃ on nOMV surface did not impact on the ability of nOMV to induce specific anti-OAg IgG response (Figure 1B).

### EXAMPLE 6: fHbp v1.1 linked to S. Typhimurium nOMV by SH-malemido or click chemistry

### Example 6.1: S. Typhimurium nOMV derivatization with SH linker.

nOMV were suspended in 100 mM borate buffer pH 8 and added of the activation buffer containing 2.6 mg/mL DTT, 13.16 mg/mL EDTA and 7.04 mg/mL N-acetyl-DL-homocysteine thiolactone linker in 100 mM borate buffer pH 11. nOMV were at 3.0 mg/mL with a molar ratio of linker to NH₂ groups on nOMV equal to 6.63. The reaction was left mixing at RT for 4 hours and nOMV-SH were then purified by dialysis against 50 mM MES 0.5 mM DTT buffer pH 6. nOMV-SH were characterised by Lowry (75% protein recovery) and TNBS showing that 41% of NH₂ groups were activated.

### Example 6.2: fHbp v1.1 derivatization with N-ε-malemidocaproyl-oxysuccinimde ester (EMCS) linker.

fHbp at the concentration of 1.25 mg/mL in PBS was added of EMCS linker (as a 10 mg/mL solution in DMSO) to have a 0.2:1 molar ratio of linker to Lys residues of the protein. The solution was mixed at RT for 4.5 hours and then the derivatised protein was purified by PD10 column against MES 10 mM pH 6. The resulting product was characterised by micro BCA (95% recovery), HPLC-SEC and SDS-PAGE showing no protein aggregation respect to underivatised protein and MALDI-MS indicating an average of three linkers introduced per molecule of protein.

### Example 6.3: Conjugation of nOMV-SH with fHbp-EMCS.

fHbp-EMCS was conjugated to nOMV-SH. Conjugation was performed with 2:1 w/w nOMV to fHbp ratio at fHbp concentration of 2 mg/mL. The reaction was left mixing at RT for 5 hours and the conjugate was purified by Vivaspin 100k against PBS. Conjugate formation was confirmed by SDS page/western blot analysis.

### Example 6.4: S. Typhimurium nOMV derivatisation with alkyne linker.

nOMV were suspended in 100 mM NaPi pH 7.2 and added of click-easy BCN N-hydroxysuccinimidester I (Berry Associates) linker 25 mg/mL in DMSO. nOMV were at 8.9 mg/mL with a molar ratio of linker to NH₂ groups on GMMA equal to 0.6. The reaction was left mixing at RT for 4 hours and nOMV-alkyne were then purified by dialysis against NaPi 100 mM pH 7.2. nOMV-alkyne were characterised by Lowry (72% protein recovery) and TNBS showing that 40% of NH₂ groups were activated.

### Example 6.5: fHbp v1.1 derivatisation with NHS-PEG₄-N₃ linker.

fHbp at the concentration of 1.25 mg/mL in PBS was added of -N₃ linker (as a 25 mg/mL solution in DMSO) to have a 0.2:1 molar ratio of linker to Lys residues of the protein. The solution was mixed at RT for 6 hours and then the derivatised protein was purified by PD10 column against NaPi 10 mM pH 7.2. The resulting product was characterised by micro BCA (87% recovery), HPLC-SEC and SDS-PAGE, showing no protein aggregation respect to the underivatised protein, and MALDI-MS, indicating an average of two linkers introduced per molecule of protein.

### Example 6.6: Conjugation of nOMV-alkyne with fHbp-N₃.

fHbp-N₃ was conjugated to nOMV-alkyne. Conjugation was performed with 2:1 w/w nOMV to fHbp ratio at fHbp concentration of 1.67 mg/mL. The reaction was left mixing at RT for 5 hours and the conjugate was purified by Vivaspin 100k against PBS. Conjugate formation was verified by SDS page/western blot analysis.

### EXAMPLE 7: immunogenicity of conjugates obtained in Example 6 in mice

Mice were immunised subcutaneously at days 0 and 28 with the synthesized conjugates compared to fHbp alone, nOMV alone and fHbp physically mixed with nOMV. 2.5 µg total protein (1.75 µg nOMV and 0.75 µg fHbp, assuming a fHbp to total protein w/w ratio in the conjugates of 0.3) per dose was used. All antigens were formulated with Alhydrogel 0.7 mg/mL Al³⁺ and 10 mM Histidine.

Antibodies induced by the conjugates showed broad protection against different meningococcal strains, with bactericidal activity higher compared with recombinant fHbp alone or physically mixed with nOMV (Table 3). Sera from conjugates also resulted bactericidal against the invasive Malawian clinical isolate D23580 S. Typhimurium strain and not inferior to nOMV alone in terms of anti-OAg IgG response induced (Table 3).

**Table 3. SBA titers against different meningococcal strains of pooled sera collected after immunization with fHbp constructs. Mice were immunized at day 0 and 28 with 2.5 µg total protein (1. 75 µg nOMV and 0.75 µg fHbp, assuming a fHbp to total protein w/w ratio in the conjugates of 0.3) formulated with Alhydrogel.**

| | **SBA titers against strains** | | | | | | | |
|---|---|---|---|---|---|---|---|---|
| | **Niga 16/09** | **Bufa 20030020** | **Cam2_09** | **Mali 4/11** | **B H44/76** | | **B M6190** | **D23580** |
| | **fHbp ID 5** | | **fHbp ID 9** | | **fHbp ID 1** | | | **STm** |
| **Sera** | **d42** | **d42** | **d42** | **d42** | **d14** | **d42** | **d42** | **d42** |
| **nOMV-SH-fHbp** | 32000 | 3600 | 2800 | <10 | 1000 | >163840 | >163840 | 66000 |
| **nOMV-click-fHbp** | 40000 | 7000 | 600 | <10 | 2800 | >163840 | 130000 | 17370 |
| **nOMV + fHbp** | 1800 | <10 | <10 | <10 | 40 | >163840 | 10000 | 365732 |
| **fHbp** | 2500 | <10 | <10 | <10 | <10 | >163840 | 2000 | <100 |
| **nOMV** | <10 | <10 | <10 | <10 | <10 | <10 | <10 | 73309 |

### EXAMPLE 8: MenB nOMV conjugated to fHbp-SH v3 protein by BS3 chemistry (no aggregates formation after conjugation)

MenB nOMV, at a protein concentration of 2.8 mg/mL in 100 mM MES buffer pH 6, were added of BS3 linker (50 mg/mL in the reaction mixture). The mixture was incubated at the controlled temperature of 25°C for 30 minutes. After this time, activated nOMV were purified by ultracentrifuge (110000 rpm, 16 min, 4°C) and immediately added of fHbp-SH v3. In the conjugation step, a 1:1 w/w ratio of nOMV to fHbp-SH was used, with nOMV concentration of 1.7 mg/ml in PBS. After overnight mixing at RT, the conjugate was purified by ultracentrifuge (110000 rpm, 16 min, 4°C) and recovered in PBS. Characterization by SDS page/western blot confirmed conjugate formation. nOMV-BS3 and final conjugate were compared to native nOMV by HPLC-SEC/MALS showing no aggregate formation (Table below).

| **Sample** | **Rw (nm)** |
|---|---|
| MenB nOMV | 44.1 |
| MenB nOMV-BS3 | 58.6 |
| MenB nOMV-BS3-fHbp conj | 54.0 |

### EXAMPLE 8a (comparative): dOMV conjugation via BS3 linker

dOMV (from MenB) has been tested as starting material for reaction with BS3 linker, under the following conditions:
- pH: 6.5;
- BS3 concentration: 50 mg/mL;
- dOMV concentration: 1.011 mg/mL;
- 30 min reaction time at 25° C;
- Purification by UC (110Krpm, 16 min, 4°C).

The reaction provides dOMV aggregates and side products as major results. Aggregation/crosslinking has been verified by dls analysis and SEC/MALS.

### EXAMPLE 9: S. Typhimurium nOMV conjugated to GAS-PS

GAS PS was terminally derivatized with ADH linker before conjugation to nOMV. The PS was solubilised in AcONa 20 mM pH 4.5 at 30 mg/mL and added of ADH and NaBH₃CN both with a 1:1 w/w ratio respect to the PS. The solution was mixed at 30°C for 5 days. After this time the product was purified by 2 PD10 column against NaCl 3 M and water, respectively. PS-ADH was characterised by HPAEC-PAD (78% recovery) and TNBS, finding that all the PS chains were activated with ADH linker.

nOMV, at a protein concentration of 4.4 mg/mL in 100 mM MES buffer pH 6, were added of BS3 linker at 50 mg/mL in the reaction mixture. The mixture was incubated at the controlled temperature of 25°C for 30 minutes. After this time, activated nOMV were purified by ultracentrifuge (110000 rpm, 16 min, 4°C) and immediately added of GAS PS-ADH. In the conjugation step, a 1:3 ratio of nOMV to GAS PS-ADH was used, with GAS PS-ADH concentration of 10 mg/ml in PBS. After overnight incubation at RT, the conjugate was purified by ultracentrifuge (110000 rpm, 16 min, 4°C) and recovered in PBS. Characterization by micro BCA, HPAEC-PAD and NTA allowed to estimate an average of 107 PS chains per particle of nOMV.

Same conjugation protocol was applied to conjugate synthetic oligosaccharides of formula (Rha)₄-(CH₂)₃-NH₂ and (Rha)₆-(CH₂)₃-NH₂ to nOMV. Resulting conjugates had an average number of 876 and 640 oligosaccharide chains per nOMV particle.

### EXAMPLE 10: in vivo data of the conjugates of the invention obtained by conjugating a S. Typhimurium nOMV particle to Pfs25 or fHbp v2 antigens by BS3 chemistry.

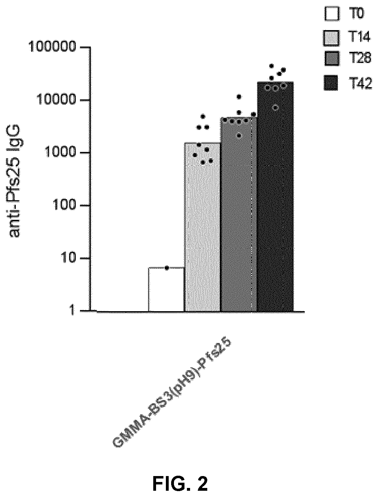

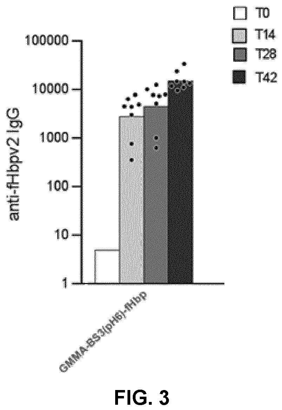

CD1 female mice were immunised subcutaneously at days 0 and 28 with 2.5 µg total protein S. Typhimurium nOMV particles conjugated to Pfs25 or fHbp antigens by BS3 chemistry. All the antigens were adsorbed on Alhydrogel (0.7 mg/mL Al³⁺). Anti-Pfs25 (Figure 2) and anti-fHbp v2 (Figure 3) IgG titers were measured at days 0, 14, 27 and 42.

As demonstrated, nOMV-BS3 conjugates were able to induce high anti-antigen specific IgG response.

### EXAMPLE 11: preparation of MenA-GMMA-MenC conjugates via SIDEA likers.

Meningococcal B GMMA overexpressing fHbp, at a protein concentration of 10 mg/mL in 50 mM NaH₂PO₄ buffer pH 7.2, were simultaneously added to MenA and MenC saccharides (avDP 15), previously terminally derivatised with SIDEA linker (Vaccine 1992 10(10):691-698), with a w/w MenA/MenC to nOMV ratio of 10:1, according to procedures know in the art. The mixture was incubated at room temperature overnight. After this time, resulting conjugate was purified by ultracentrifuge (2x, 110000 rpm, 1h, 4°C) and suspended in PBS. Analysis by SDS PAGE western blot confirmed conjugate formation, with both saccharides linked to nOMV. The conjugate was characterised by a w/w MenA to protein ratio and MenC to protein ratio of 0.10 and 0.12 respectively, as determined by HPAEC-PAD and micro BCA analysis, with 19.3% of MenA and 4.3% of MenC free saccharide respectively (by HPAEC-PAD analysis after C4 SPE treatment of the conjugates).

### EXAMPLE 12: preparation of MenA-GMMA conjugates via SIDEA linker

Meningococcal B GMMA overexpressing fHbp, at a protein concentration of 10 mg/mL in 50 mM NaH₂PO₄ buffer pH 7.2, were added to MenA polysaccharide (avDP 15), previously terminally derivatised with SIDEA linker (Vaccine 1992 10(10):691-698), with a w/w MenA to nOMV ratio of 10:1, according to procedures know in the art. The mixture was incubated at room temperature overnight. After this time, resulting conjugate was purified by ultracentrifuge (2x, 110000 rpm, 1h, 4°C) and suspended in PBS. Analysis by SDS PAGE western blot confirmed conjugate formation. The conjugate was characterised by a w/w MenA to protein ratio of 0.054, as determined by HPAEC-PAD and micro BCA analysis, with no free MenA detected by HPAEC-PAD analysis after C4 SPE treatment of the conjugate.

### EXAMPLE 13: preparation of MenC-GMMA conjugates via SIDEA linker

The same procedure used in Example 12 has been followed for the preparation of the titled conjugate, using an antigen from MenC in place of MenA. Conjugate characterization is reported in the Table below.

| **Conjugate** | **OS/GMMA w/w ratio** | **Number OS per GMMA** | **µg/mL OS** | **µg/mL GMMA** | **% free OS** |
|---|---|---|---|---|---|
| GMMA-MenA | 0.054 | 2490 | 102.1 | 1901.6 | not detected |
| GMMA-MenC | 0.1 | 4523 | 195.1 | 1942.6 | not detected |
| Bivalent MenC-GMMA-MenA | 0.103 (MenA) | 3847 (MenA) | 151.3 (MenA) | 1468.2 | 4.3% free MenC OS, 19.3% free MenA OS |
| | 0.12 (MenC) | 5168 (MenC) | 176.3 (MenC) | | |

### EXAMPLE 14: immunogenicity study in mice

CD1 female mice 5-6 weeks old (8 per group) were immunised intramuscolarly at days 0 and 28 with 1 µg MenA or MenC per dose. GMMA conjugates were compared with MenA-CRM₁₉₇ or MenC-CRM₁₉₇ conjugates, and with GMMA physically mixed to MenA or MenC polysaccharides.

All the antigens were adsorbed on Alhydrogel (0.7 mg/mL Al³⁺). Anti-MenA and MenC IgG titers were measured at days -1, 27 and 42 (Figure 2) and SBA titers of pooled sera from each group at day 42 were measured against a panel of MenA, MenC and MenB strains.

### EXAMPLE 14a: Anti-MenA IgG/SBA

### IgG response

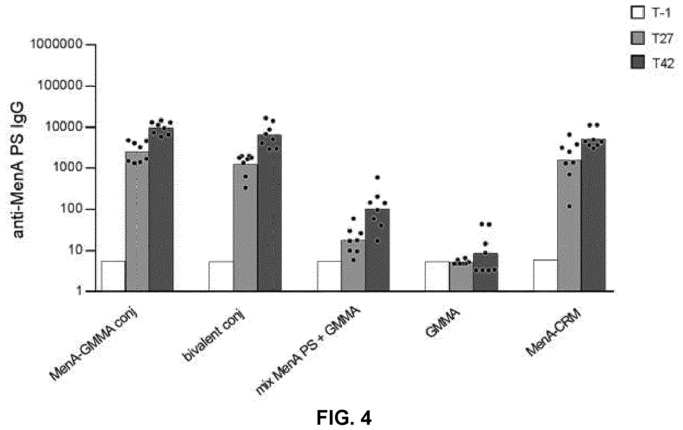

As showed in the Figure 4, the immunogenicity study in mice showed that:
- Higher anti-MenA IgG response is induced by MenA-GMMA conjugate compared to MenA-CRM₁₉₇
- Conjugation of MenA to GMMA enhances the humoral immune response against the polysaccharide.
- The bivalent conjugate induces anti-MenA IgG response comparable to MenA-GMMA conjugate, with no interference due to MenC presence on the same GMMA particles.

### SBA against MenA

| **Antigen** | **MenA-GMMA conj** | **Bivalent conj** | **MenA PS + GMMA** | **GMMA** | **MenA-CRM₁₉₇** |
|---|---|---|---|---|---|
| rSBA MenA strain F8238 | 262144 | 65536 | 1024 | <16 | 8192 |

### EXAMPLE 14b: Anti-MenC IgG/SBA

### Conjugates tested in mice

### IgG response

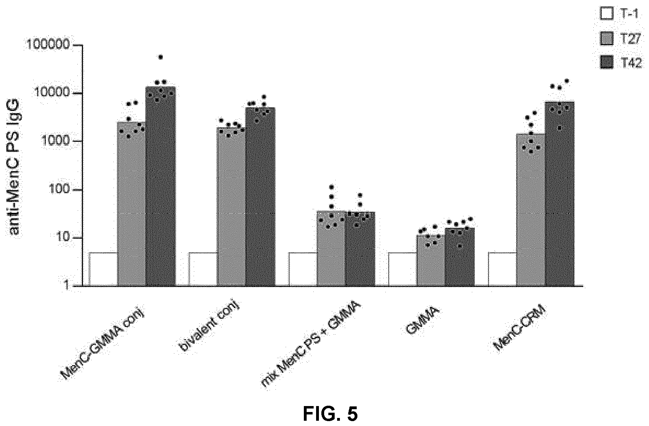

As showed in Figure 5, the immunogenicity study in mice showed that:
- Similar anti-MenC IgG response induced by MenC-GMMA compared to MenC-CRM₁₉₇
- Conjugation of MenC to GMMA enhances the humoral immune response against OS

### SBA against MenC

| **Antigen** | **MenA-GMMA conj** | **Bivalent conj** | **MenA PS + GMMA** | **GMMA** | **MenA-CRM₁₉₇** |
|---|---|---|---|---|---|
| rSBA MenC strain C11 | 524288 | 262144 | 8192 | 4096 | 32768 |

### EXAMPLE 14c: Anti-MenB SBA

### SBA against MenB for GMMA conjugated to MenA and/or MenC

| **Antigen** | **MenB strain UK 320** | **MenB strain UK 104** |
|---|---|---|
| GMMA-MenA conjugate | 16384 | 32768 |
| GMMA-MenC conjugate | 32768 | 16384 |
| Bivalent conjugate | 1024 | 1024 |
| GMMA | 131072 | 65536 |
| MenA-CRM | <16 | <16 |
| MenC-CRM | <16 | <16 |

The above data show that the conjugation of one or more antigens to the same GMMA according to the invention still provide very good immunogenic profile of the antigens or the GMMA. This means that the present conjugates do not show interference of the antigens, confirming the potential use of the conjugates for the preparation of polyvalent immunogenic composition or vaccine against a variety of pathogens.

### SEQUENCE LISTING

>SEQ ID NO: 1 [fHbp v2]
VAADIGAGLADALTAPLDHKDKSLQSLTLDQSVRKNEKLKLAAQGAEKTYGNGDSLNTGKLKN
DKVSRFDFIRQIEVDGQLITLESGEFQIYKQDHSAVVALQIEKINNPDKIDSLINQRSFRVSGLGG
EHTAFNQLPDGKAEYHGKAFSSDDAGGKLTYTIDFAAKQGHGKIEHLKTPEQNVELAAAELKA
DEKSHAVILGDTRYGSEEKGTYHLALFGDRAQEIAGSATVKIGEKVHEIGIAGKQ

>SEQ ID NO: 2 [NHBA]
MFKRSVIAMACIFALSACGGGGGGSPDVKSADTLSKPAAPVVSEKETEAKEDAPQAGSQGQG
APSAQGSQDMAAVSEENTGNGGAVTADNPKNEDEVAQNDMPQNAAGTDSSTPNHTPDPNM
LAGNMENQATDAGESSQPANQPDMANAADGMQGDDPSAGGQNAGNTAAQGANQAGNNQA
AGSSDPIPASNPAPANGGSNFGRVDLANGVLIDGPSQNITLTHCKGDSCSGNNFLDEEVQLKS
EFEKLSDADKISNYKKDGKNDKFVGLVADSVQMKGINQYIIFYKPKPTSFARFRRSARSRRSLP
AEMPLIPVNQADTLIVDGEAVSLTGHSGNIFAPEGNYRYLTYGAEKLPGGSYALRVQGEPAKGE
MLAGAAVYNGEVLHFHTENGRPYPTRGRFAAKVDFGSKSVDGIIDSGDDLHMGTQKFKAAIDG
NGFKGTWTENGSGDVSGKFYGPAGEEVAGKYSYRPTDAEKGGFGVFAGKKEQD

>SEQ ID NO: 3 [NadA]

MKHFPSKVLTTAILATFCSGALAATSDDDVKKAATVAIVAAYNNGQEINGFKAGETIYDIGEDGTI
TQKDATAADVEADDFKGLGLKKVVTNLTKTVNENKQNVDAKVKAAESEIEKLTTKLADTDAALA
DTDAALDETTNALNKLGENITTFAEETKTNIVKIDEKLEAVADTVDKHAEAFNDIADSLDETNTKA
DEAVKTANEAKQTAEETKQNVDAKVKAAETAAGKAEAAAGTANTAADKAEAVAAKVTDIKADIA
TNKADIAKNSARIDSLDKNVANLRKETRQGLAEQAALSGLFQPYNVGRFNVTAAVGGYKSESA
VAIGTGFRFTENFAAKAGVAVGTSSGSSAAYHVGVNYEW

>SEQ ID NO: 4 [NspA]
MKKALATLIALALPAAALAEGASGFYVQADAAHAKASSSLGSAKGFSPRISAGYRINDLRFAVDY
TRYKNYKAPSTDFKLYSIGASAIYDFDTQSPVKPYLGARLSLNRASVDLGGSDSFSQTSIGLGVL
TGVSYAVTPNVDLDAGYRYNYIGKVNTVKNVRSGELSAGVRVKF

>SEQ ID NO: 5 [NhhA]
MNKIYRIIWNSALNAWVVVSELTRNHTKRASATVKTAVLATLLFATVQASANNEEQEEDLYLDP
VQRTVAVLIVNSDKEGTGEKEKVEENSDWAVYFNEKGVLTAREITLKAGDNLKIKQNGTNFTYS
LKKDLTDLTSVGTEKLSFSANGNKVNITSDTKGLNFAKETAGTNGDTTVHLNGIGSTLTDTLLNT
GATTNVTNDNVTDDEKKRAASVKDVLNAGWNIKGVKPGTTASDNVDFVRTYDTVEFLSADTKT
TTVNVESKDNGKKTEVKIGAKTSVIKEKDGKLVTGKDKGENGSSTDEGEGLVTAKEVIDAVNKA
GWRMKTTTANGQTGQADKFETVTSGTNVTFASGKGTTATVSKDDQGNITVMYDVNVGDALNV
NQLQNSGWNLDSKAVAGSSGKVISGNVSPSKGKMDETVNINAGNNIEITRNGKNIDIATSMTPQ
FSSVSLGAGADAPTLSVDGDALNVGSKKDNKPVRITNVAPGVKEGDVTNVAQLKGVAQNLNN
RIDNVDGNARAGIAQAIATAGLVQAYLPGKSMMAIGGGTYRGEAGYAIGYSSISDGGNWIIKGT
ASGNSRGHFGASASVGYQW

>SEQ ID NO: 6 [App]
MKTTDKRTTETHRKAPKTGRIRFSPAYLAICLSFGILPQAWAGHTYFGINYQYYRDFAENKGKF
AVGAKDIEVYNKKGELVGKSMTKAPMIDFSVVSRNGVAALVGDQYIVSVAHNGGYNNVDFGAE
GRNPDQHRFTYKIVKRNNYKAGTKGHPYGGDYHMPRLHKFVTDAEPVEMTSYMDGRKYIDQN
NYPDRVRIGAGRQYWRSDEDEPNNRESSYHIASAYSWLVGGNTFAQNGSGGGTVNLGSEKIK
HSPYGFLPTGGSFGDSGSPMFIYDAQKQKWLINGVLQTGNPYIGKSNGFQLVRKDWFYDEIFA
GDTHSVFYEPRQNGKYSFNDDNNGTGKINAKHEHNSLPNRLKTRTVQLFNVSLSETAREPVYH
AAGGVNSYRPRLNNGENISFIDEGKGELILTSNINQGAGGLYFQGDFTVSPENNETWQGAGVHI
SEDSTVTWKVNGVANDRLSKIGKGTLHVQAKGENQGSISVGDGTVILDQQADDKGKKQAFSEI
GLVSGRGTVQLNADNQFNPDKLYFGFRGGRLDLNGHSLSFHRIQNTDEGAMIVNHNQDKEST
VTITGNKDIATTGNNNSLDSKKEIAYNGWFGEKDTTKTNGRLNLVYQPAAEDRTLLLSGGTNLN
GNITQTNGKLFFSGRPTPHAYNHLNDHWSQKEGIPRGEIVWDNDWINRTFKAENFQIKGGQAV
VSRNVAKVKGDWHLSNHAQAVFGVAPHQSHTICTRSDWTGLTNCVEKTITDDKVIASLTKTDIS

GNVDLADHAHLNLTGLATLNGNLSANGDTRYTVSHNATQNGNLSLVGNAQATFNQATLNGNT
SASGNASFNLSDHAVQNGSLTLSGNAKANVSHSALNGNVSLADKAVFHFESSRFTGQISGGKD
TALHLKDSEWTLPSGTELGNLNLDNATITLNSAYRHDAAGAQTGSATDAPRRRSRRSRRSLLS
VTPPTSVESRFNTLTVNGKLNGQGTFRFMSELFGYRSDKLKLAESSEGTYTLAVNNTGNEPAS
LEQLTVVEGKDNKPLSENLNFTLQNEHVDAGAWRYQLIRKDGEFRLHNPVKEQELSDKLGKAE
AKKQAEKDNAQSLDALIAAGRDAVEKTESVAEPARQAGGENVGIMQAEEEKKRVQADKDTALA
KQREAETRPATTAFPRARRARRDLPQLQPQPQPQPQRDLISRYANSGLSEFSATLNSVFAVQD
ELDRVFAEDRRNAVWTSGIRDTKHYRSQDFRAYRQQTDLRQIGMQKNLGSGRVGILFSHNRT
ENTFDDGIGNSARLAHGAVFGQYGIDRFYIGISAGAGFSSGSLSDGIGGKIRRRVLHYGIQARYR
AGFGGFGIEPHIGATRYFVQKADYRYENVNIATPGLAFNRYRAGIKADYSFKPAQHISITPYLSLS
YTDAASGKVRTRVNTAVLAQDFGKTRSAEWGVNAEIKGFTLSLHAAAAKGPQLEAQHSAGIKL
GYRW

>SEQ ID NO: 7 [NadA fragment]
ATNDDDVKKAATVAIAAAYNNGQEINGFKAGETIYDIDEDGTITKKDATAADVEADDFKGLGLKK
VVTNLTKTVNENKQNVDAKVKAAESEIEKLTTKLADTDAALADTDAALDATTNALNKLGENITTF
AEETKTNIVKIDEKLEAVADTVDKHAEAFNDIADSLDETNTKADEAVKTANEAKQTAEETKQNVD
AKVKAAETAAGKAEAAAGTANTAADKAEAVAAKVTDIKADIATNKDNIAKKANSADVYTREESD
SKFVRIDGLNATTEKLDTRLASAEKSIADHDTRLNGLDKTVSDLRKETRQGLAEQAALSGLFQP
YNVG

>SEQ ID NO: 8 [fHbp v1]
VAADIGAGL ADALTAPLDH KDKGLQSLTL DQSVRKNEKL KLAAQGAEKT YGNGDSLNTG
KLKNDKVSRF DFIRQIEVDG QLITLESGEF QVYKQSHSAL TAFQTEQIQD SEHSGKMVAK
RQFRIGDIAG EHTSFDKLPE GGRATYRGTA FGSDDAGGKL TYTIDFAAKQ GNGKIEHLKS
PELNVDLAAA DIKPDGKRHA VISGSVLYNQ AEKGSYSLGI FGGKAQEVAG SAEVKTVNGI
RHIGLAAKQ

>SEQ ID NO: 9 [fHbp v3]
VAADIGTGLADALTAPLDHKDKGLKSLTLEDSIPQNGTLTLSAQGAEKTFKAGDKDNSLNTGKL
KNDKISRFDFVQKIEVDGQTITLASGEFQIYKQNHSAVVALQIEKINNPDKTDSLINQRSFLVSGL
GGEHTAFNQLPGGKAEYHGKAFSSDDPNGRLHYSIDFTKKQGYGRIEHLKTLEQNVELAAAEL
KADEKSHAVILGDTRYGSEEKGTYHLALFGDRAQEIAGSATVKIGEKVHEIGIAGKQ

>SEQ ID NO: 10 [Pfs25]
KVTVDTVCKR GFLIQMSGHL ECKCENDLVL VNEETCEEKV LKCDEKTVNK PCGDFSKCIK
IDGNPVSYAC KCNLGYDMVN NVCIPNECKQ VTCGNGKCIL DTSNPVKTGV CSCNIGKVPN
VQDQNKCSKD GETKCSLKCL KEQETCKAVD GIYKCDCKDG FIIDQESSIC T

>SEQ ID NO: 11 [RO6C]
AERSTSENRNKRIGGPKLRGNVTSNIKFPSDNKGKIIRGSNDKLNKNSEDVLEQSEKSLVSENV
PSGLDIDDIPKESIFIQEDQEGQTHSELNPETSEHSKDLNNNGSKNESSDIISENNKSNKVQNHF
ESLSDLELLENSSQDNLDKDTISTEPFPNQKHKDLQQDLNDEPLEPFPTQIHKDYKEKNLINEED
SEPFPRQKHKKVDNHNEEKNVFHENGSANGNQGSLKLKSFDEHLKDEKIENEPLVHENLSIPN
DPIEQILNQPEQETNIQEQLYNEKQNVEEKQNSQIPSLDLKEPTNEDILPNHNPLENIKQSESEIN
HVQDHALPKENIIDKLDNQKEHIDQSQHNINVLQENNINNHQLEPQEKPNIESFEPKNIDSEIILPE
NVETEEIIDDVPSPKHSNHETFEEETSESEHEEAVSEKNAHETVEHEETVSQESNPEKADNDG
NVSQNSNNELNENEFVESEKSEHEARSKPKYEKKVIHGCNFSSNVSSKHTFTDSLDISLVDDSA
HISCNVHLSEPKYNHLVGLNCPGDIIPDCFFQVYQPESEELEPSNIVYLDSQINIGDIEYYEDAEG
DDKIKLFGIVGSIPKTTSFTCICKKDKKSAYMTVTIDSARSHHHHHH

>SEQ ID NO: 12 [CSP]
MLFQEYQCYGSSSNTRVLNELNYDNAGTNLYNELEMNYYGKQENWYSLKKNSRSLGENDDG
NNNNGDNGREGKDEDKRDGNNEDNEKLRKPKHKKLKQPGDGNPDPNANPNVDPNANPNVD
PNANPNVDPNANPNANPNANPNANPNANPNANPNANPNANPNANPNANPNANPNANPNANP
NANPNANPNVDPNANPNANPNANPNANPNANPNANPNANPNANPNANPNANPNANPNANPN

ANPNANPNANPNANPNANPNANPNANPNKNNQGNGQGHNMPNDPNRNVDENANANNAVKN
NNNEEPSDKHIEKYLKKIQNSLSTEWSPCSVTCGNGIQVRIKPGSANKPKDELDYENDIEKKICK
MEKCSSVFNVVNSSIGLILEHHHHHH

**>SEQ ID NO: 13 [(NANP)3]**
NANPNANPNANP

>SEQ ID NO: 14 [CTF1232]
QDQRYISIRNTDTIWLPGNICAYQFRLDNGGNDEGFGPLTITLQLKDKYGQTLVTRKMETEAFG
DSNATRTTDAFLETECVENVATTEIIKATEESNGHRVSLPLSVFDPQDYHPLLITVSGKNVNLEH
HHHHH

>SEQ ID NO: 15 [3526, SslE]
DTPSVDSGSGTLPEVKPDPTPTPEPTPEPTPDPEPTPDPTPDPEPTPEPEPEPVPTKTGYLTLG
GSQRVTGATCNGESSDGFTFTPGNTVSCVVGSTTIATFNTQSEAARSLRAVDKVSFSLEDAQE
LANSENKKTNAISLVTSSDSCPADAEQLCLTFSSVVDRARFEKLYKQIDLATDNFSKLVNEEVEN
NAATDKAPSTHTSTVVPVTTEGTKPDLNASFVSANAEQFYQYQPTEIILSEGQLVDSLGNGVAG
VDYYTNSGRGVTDENGKFSFSWGETISFGIDTFELGSVRGNKSTIALTELGDEVRGANIDQLIH
RYSTTGQNNTRVVPDDVRKVFAEYPNVINEIINLSLSNGATLDEGDQNVVLPNEFIEQFKTGQA
KEIDTAICAKTDGCNEARWFSLTTRNVNDGQIQGVINKLWGVDTNYQSVSKFHVFHDSTNFYG
STGNARGQAVVNISNSAFPILMARNDKNYWLAFGEKRAWDKNELAYITEAPSIVQPENVT
RDTATFNLPFISLGQVGEGKLMVIGNPHYNSILRCPNGYSWGGGVNSKGECTLSGDSDDM
KHFMQNVLRYLSNDIWQPNTKSIMTVGTNLENVYFKKAGQVLGNSAPFAFHEDFTGITVK
QLTSYGDLNPEEIPLLILNGFEYVTQWSGDPYAVPLRADTSKPKLTQQDVTDLIAYLNKG
GSVLIMENVMSNLKEESASSFVRLLDAAGLSMALNKSVVNNDPQGYPDRVRQRRATGIWV
YERYPAADGAQPPYTIDPNTGEVTWKYQQDNKPDDKPKLEVASWQEEVEGKQVTRYAFID
EAEYTTEESLEAAKAKIFEKFPGLQECKDSTYHYEINCLERRPGTDVPVTGGMYVPRYTQ
LNLDADTAKAMVQAADLGTNIQRLYQHELYFRTKGSKGERLNSVDLERLYQNMSVWLWND
TKYRYEEGKEDELGFKTFTEFLNCYANDAYAGGTKCSADLKKSLVDNNMIYGDGSSKAGM
MNPSYPLNYMEKPLTRLMLGRSWWDLNIKVDVEKYPGSVSAKGESVTENISLYSNPTKWF
AGNMQSTGLWAPAQQDVTIKSSASVPVTVTVALADDLTGREKHEVALNRPPRVTKTYTLE
ANGEVTFKVPYGGLIYIKGDSKDDVSANFTFTGVVKAPFYKDGEWKNDLDSPAPLGELES
ASFVYTTPKKNLEASNFTGGVAEFAKDLDTFASSMNDFYGRNDEDGKHRMFTYKNLTGHK
HRFTNDVQISIGDAHSGYPVMNSSFSTNSTTLPTTPLNDWLIWHEVGHNAAETPLNVPGA
TEVANNVLALYMQDRYLGKMNRVADDITVAPEYLDESNGQAWARGGAGDRLLMYAQLKEW
AEENFDIKQWYPDGELPKFYSDRKGMKGWNLFQLMHRKARGDDVGNSTFGGKNYCAESNG
NAADTLMLCASWVAQADLSEFFKKWNPGASAYQLPGATEMSFQGGVSSSAYSTLASLKLP
KPEKGPETINKVTEHKMSAE

>SEQ ID NO: 16 [405, FdeC]
VADGQQAYTLTLTAVDSEGNPVTGEASRLRLVPQDTNGVTVGAISEIKPGVYSATVSSTR
AGNVVVRAFSEQYQLGTLQQTLKFVAGPLDAAHSSITLNPDKPVVGGTVTAIWTAKDAND
NPVTGLNPDAPSLSGAAAAGSTASGWTDNGDGTWTAQISLGTTAGELDVMPKLNGQDAAA
NAAKVTVVADALSSNQSKVSVAEDHVKAGESTTVTLVAKDAHGNAISGLSLSASLTGTAS
EGATVSSWTEKGDGSYVATLTTGGKTGELRVMPLFNGQPAATEAAQLTVIAGEMSSANST
LVADNKTPTVKTTTELTFTMKDAYGNPVTGLKPDAPVFSGAASTGSERPSAGNWTEKGNG
VYVSTLTLGSAAGQLSVMPRVNGQNAVAQPLVLNVAGDASKAEIRDMTVKVNNQ

### EMBODIMENTS OF THE INVENTION

**1.** An immunogenic conjugate comprising a native Outer Membrane Vesicle (nOMV) having at least a surface protein moiety connected to at least an antigen by a divalent linker.
**2.** The immunogenic conjugate according to embodiment 1, comprising a nOMV having at least a surface protein moiety connected to a first antigen by a divalent linker, wherein said first antigen is further connected to a second different antigen.
**3.** The immunogenic conjugate according to embodiment 1, comprising a nOMV having at least a surface protein moiety connected to a first antigen by a divalent linker, and at least another surface protein moiety connected to a second different antigen by a divalent linker.
**4.** The immunogenic conjugate according to the preceding embodiments, wherein said divalent linker has the general formula (I):

X-L-X' **(I)**

wherein:
- X and X': are different to each other or the same, and are a functional group able to selectively react with the nOMV protein residue on one hand and with the antigen on the other hand;
- -L-: is a divalent linear or branched C₁-C₁₅ alkyl or alkenyl group optionally substituted, and optionally interrupted by one or more heteroatom selected from: oxygen (-O-), sulfur (-S-), nitrogen (-NH- or optionally substituted -N- group) and the like.
**5.** The immunogenic conjugate according to embodiment 4, wherein said divalent linker is a homobifunctional linker having X = X'.
**6.** The immunogenic conjugate according to embodiment 4, wherein said divalent linker is selected from at least one of: disuccinimidyl glutarate (DSG), disuccinimidyl suberate (DSS), succinimidyl 3-(2-pyridyldithio)propionate (SPDP), Succinimidyl 6-(3-[2-pyridyldithio]-propionamido)hexanoate (LC-SPDP), sulfosuccinimidyl 6-(3'-(2-pyridyldithio)propionamido)hexanoate (sulfo-LC-SPDP), 4-succinimidyloxycarbonyl-α-methyl-α-(2-pyridyldithio)toluene (SMPT), sulfosuccinimidyl-6-[α-methyl-α-(2-pyridyldithio)tolueamideo]hexanoate (sulfo-LC-SMPT), succinimidyl 4-(N-maleimidomethyl)cyclohexane-1-carboxylate (SMCC), sulfosuccinimidyl 4-(N-maleimidomethyl)cyclohexane-1-carboxylate (suflo-SMCC), m-maleimidobenzoyl-N-hydroxysuccinimide ester (MBS), m-maleimidobenzoyl-N-hydroxysulfosuccinimide ester (sulfo-MBS), N-succinimidyl (4-iodoacetyl)aminobenzoate (SIAB), sulfosuccinimidyl (4-iodoacetyl)aminobenzoate (sulfo-SIAB), succinimidyl 4-(N-maleimidophenyl)butyrate (SMPB), sulfosuccinimidyl 4-(N-maleimidophenyl)butyrate(sulfo-SMPB), N-γ-maleimidobutyryl-oxysuccinimide ester (GMBS), N-γ-maleimidobutyryl-oxysulfosuccinimide ester (sulfo-GMBS), succinimidyl-6-((((4-(iodoacetyl)amino)methyl)cyclohexane-1-carbonyl)amino)hexanoate (SIACX), succinimidyl 6[6-(((iodoacetyl)amino)hexanoyl)amino]hexanoate (SIAXX), succinimidyl-4-(((iodoacetyl)amino)methyl)cyclohexane-1-carboxylate (SIAC), succinimidyl 6-[(iodoacetyl)amino]hexanoate (SIAX) and p-nitrophenyl iodoacetate (NPIA), N-hydroxysuccinimide, N oxysuccinimide and adipic acid N-hydroxysuccinimide diester (SIDEA) and Bis(sulfosuccinimidyl) suberate (BS3), acryloyl halides (e.g. chloride), ethylene glycol bis[succinimidylsuccinate], bis(sulfosuccinimidyl)tri(ethylene glycol) (BS(PEG)3), bis(sulfosuccinimidyl)tetra(ethylene glycol) (BS(PEG)4), bis(sulfosuccinimidyl)penta(ethylene glycol) (BS(PEG)5) and bis(sulfosuccinimidyl)exa(ethylene glycol) (BS(PEG)6), adipic acid dihydrazide (ADH), β-propionamido, nitrophenyl-ethylamine, haloacyl halides, and 6-aminocaproic acid.
**7.** The immunogenic conjugate according to embodiment 3, wherein said first antigen comprises Hib and said second agent comprises Hia, wherein said first and second antigen are each individually conjugated to a GMMA pertussis vesicle by a BS3 linker.
**8.** The immunogenic conjugate of embodiment 3, wherein said first antigen comprises ETEC 405 and said second agent comprises ETEC 3526, where said first and second agent are each individually conjugated to a GMMA Shigella Sonnei vesicle by a BS3 linker.
**9.** The immunogenic conjugate according to the preceding embodiments, wherein said nOMV is obtained by a detergent free process, being released into the fermentation broth and purified using a centrifugation and subsequent filtration; or being released into the fermentation broth and purified using two consecutive Tangential Flow Filtration (TFF) steps.
**10.** The immunogenic conjugate according to the preceding embodiments, wherein said nOMV is produced from wild type bacteria or from genetically-modified bacterial strains that are mutated to enhance vesicle production, and optionally also to remove or modify antigens and/or to over-express homologous antigens or antigens from other organisms.
**11.** The immunogenic conjugate according to the preceding embodiments, wherein said nOMV is obtained from a bacteria selected from: Neisseria, Shigella, Salmonella enterica serovars, Haemophilus influenzae, Vibrio cholerae, Bordetella pertussis, Mycobacterium smegmatis, Mycobacterium bovis BCG, Escherichia coli, Bacteroides, Pseudomonas aeruginosa, Helicobacter pylori, Brucella melitensis Campylobacter jejuni, Actinobacillus actinomycetemcomitans, Xenorhabdus nematophilus, Moraxella catarrhalis, or Borrelia burgdorferi.
**12.** The immunogenic conjugate according to embodiment 1, wherein said nOMV, divalent linker and the antigen are selected as follows:

| **nOMV** | **linker** | **Antigen** |
|---|---|---|
| *Salmonella* Typhimurium | BS3 | Plasmodium falciparum Pfs25 |
| *Salmonella* Typhimurium | BS(PEG)₅ | Plasmodium falciparum Pfs25 |
| *Salmonella* Typhimurium | BS3 | fHbp *(Neisseria meningitidis)* |
| *Salmonella* Typhimurium | BS(PEG)₅ | fHbp *(Neisseria meningitidis)* |
| *Salmonella* Typhimurium | BS3 | Plasmodium falciparum RO6C |
| *Salmonella* Typhimurium | BS(PEG)₅ | Plasmodium falciparum RO6C |
| *Salmonella* Typhimurium | BS3 | Plasmodium falciparum CSP |
| *Salmonella* Typhimurium | BS(PEG)₅ | Plasmodium falciparum CSP |
| *Meningococcal B* | BS3 | fHbp *(Neisseria meningitidis)* |
| *Meningococcal B* | BS(PEG)₅ | fHbp *(Neisseria meningitidis)* |
| *Meningococcal B* | BS3 | NHBA *(Neisseria meningitidis)* |
| *Meningococcal B* | BS(PEG)₅ | NHBA *(Neisseria meningitidis)* |
| *Salmonella* Typhimurium | BS3 | Synthetic or native GAS PS |
| *Salmonella* Typhimurium | BS(PEG)₅ | Synthetic or native GAS PS |
| *Salmonella* Typhimurium | BS3 | Synthetic or native GBS PS |
| Meningococcal B | BS3 | Synthetic or native GAS PS |
| Meningococcal B | BS3 | Hib PS |
| *B. Pertussis* | BS3 | Hib PS |
| *Salmonella* Typhimurium | BS3 | Vi PS |
| *Shigella* | BS3 | ETEC 405 |
| *Shigella* | BS3 | ETEC 3526 |
| *Shigella* | BS3 | ETEC CTF1232 |
| Meningococcal B | SIDEA | MenA *(Neisseria meningitidis)* |
| Meningococcal B | SIDEA | MenC *(Neisseria meningitidis)* |

**13.** The immunogenic conjugate according to embodiment 12 wherein said nOMV, divalent linker and the antigen are selected as follows:

| **nOMV** | **linker** | **Antigen** |
|---|---|---|
| Meningococcal B | SIDEA | MenA *(Neisseria meningitidis)* |
| Meningococcal B | SIDEA | MenC *(Neisseria meningitidis)* |

**14.** The immunogenic conjugate according to embodiment 3, comprising a nOMV having at least a surface protein moiety connected to a first antigen by a divalent linker which is SIDEA, and at least another surface protein moiety connected to a second different antigen by a divalent linker which is SIDEA or BS3.
**15.** The immunogenic conjugate according to embodiment 3, comprising a nOMV having at least a surface protein moiety connected to a first antigen by a divalent linker which is BS3, and at least another surface protein moiety connected to a second different antigen by a divalent linker which is BS3.
**16.** The immunogenic conjugate according to embodiments 3, 14 or 15 wherein said first antigen comprises an oligomer of *Nesseria mengitidis* serogroup C (MenC), and said second antigen comprises an oligomer of *Nesseria mengitidis* serogroup A (MenA).
**17.** The immunogenic conjugate according to embodiment 16 wherein said first antigen comprising an oligomer of *Nesseria mengitidis* serogroup C (MenC), and said second antigen comprising an oligomer of *Nesseria mengitidis* serogroup A (MenA) are each individually connected to a nOMV by a SIDEA linker.
**18.** The immunogenic conjugate according to embodiments 1-17, wherein said nOMV is a GMMA vesicle.
**19.** The immunogenic conjugate according to embodiments 1-3 and 16-17, wherein said nOMV is a GMMA vesicle obtained from *Nesseria mengitidis* serogroup B.
**20.** The immunogenic conjugate according to embodiment 19, wherein said nOMV is a GMMA vesicle obtained from Nesseria mengitidis serogroup B, preferably expressing fHbp v2 and v3.
**21.** A process for preparing the immunogenic conjugate according to embodiments 1-20, comprising the steps of:
i) reacting at least a nOMV surface protein residue with the first terminal portion of a divalent linker to obtain a nOMV-linker intermediate; and
ii) reacting said nOMV-linker intermediate with at least one antigen via the second terminal portion of the divalent linker, thus obtaining the nOMV-linker-antigen conjugate.
**22.** The nOMV-linker intermediate obtained (or obtainable) by the step i) of embodiment 21.
**23.** Use of the nOMV-linker intermediate of claim 22 for the preparation of nOMV-linker-antigen according to embodiments 1-20.
**24.** An immunogenic composition comprising a conjugate according to any one of embodiments 1-20 and at least one pharmaceutically acceptable carrier or excipient.
**25.** The immunogenic conjugate or composition according to any one of embodiments 1-20 or 24, for use as medicament.
**26.** The immunogenic conjugate or composition for use according to embodiment 25 to induce an immune response in a vertebrate.
**27.** A vaccine comprising the immunogenic conjugates of embodiments 1-20 or the immunogenic composition according to embodiment 24.
**28.** Use of nOMV for the preparation of immunogenic conjugates.
**29.** Use of nOMV according to embodiment 28, wherein said immunogenic conjugates are as defined in any one of embodiments 1-20.

## Claims

1. An immunogenic conjugate comprising a native Outer Membrane Vesicle (nOMV) having at least a surface protein moiety connected to at least an antigen by a divalent linker.

2. The immunogenic conjugate according to claim 1, comprising a nOMV having at least a surface protein moiety connected to a first antigen by a divalent linker, wherein said first antigen is further connected to a second different antigen.

3. The immunogenic conjugate according to claim 1, comprising a nOMV having at least a surface protein moiety connected to a first antigen by a divalent linker, and at least another surface protein moiety connected to a second different antigen by a divalent linker.

4. The immunogenic conjugate according to the preceding claims, wherein said divalent linker has the general formula (I):
X-L-X' (I)
wherein:
X and X' are different to each other or the same, and are a functional group able to selectively react with the nOMV protein residue on one hand and with the antigen on the other hand;
-L- is a divalent linear or branched C₁-C₁₅ alkyl or alkenyl group optionally substituted, and optionally interrupted by one or more heteroatom selected from: oxygen (-O-), sulfur (-S-), nitrogen (-NH- or optionally substituted -N- group) and the like.

5. The immunogenic conjugate according to claim 4, wherein said divalent linker is a homobifunctional linker having X = X'.

6. The immunogenic conjugate according to claim 4, wherein said divalent linker is selected from at least one of: disuccinimidyl glutarate (DSG), disuccinimidyl suberate (DSS), succinimidyl 3-(2-pyridyldithio)propionate (SPDP), Succinimidyl 6-(3-[2-pyridyldithio]-propionamido)hexanoate (LC-SPDP), sulfosuccinimidyl 6-(3'-(2-pyridyldithio)propionamido)hexanoate (sulfo-LC-SPDP), 4-succinimidyloxycarbonyl-α-methyl-α-(2-pyridyldithio)toluene (SMPT), sulfosuccinimidyl-6-[α-methyl-α-(2-pyridyldithio)tolueamideo]hexanoate (sulfo-LC-SMPT), succinimidyl 4-(N-maleimidomethyl)cyclohexane-1-carboxylate (SMCC), sulfosuccinimidyl 4-(N-maleimidomethyl)cyclohexane-1-carboxylate (suflo-SMCC), m-maleimidobenzoyl-N-hydroxysuccinimide ester (MBS), m-maleimidobenzoyl-N-hydroxysulfosuccinimide ester (sulfo-MBS), N-succinimidyl (4-iodoacetyl)aminobenzoate (SIAB), sulfosuccinimidyl (4-iodoacetyl)aminobenzoate (sulfo-SIAB), succinimidyl 4-(N-maleimidophenyl)butyrate (SMPB), sulfosuccinimidyl 4-(N-maleimidophenyl)butyrate(sulfo-SMPB), N-γ-maleimidobutyryl-oxysuccinimide ester (GMBS), N-γ-maleimidobutyryl-oxysulfosuccinimide ester (sulfo-GMBS), succinimidyl-6-((((4-(iodoacetyl)amino)methyl)cyclohexane-1-carbonyl)amino)hexanoate (SIACX), succinimidyl 6[6-(((iodoacetyl)amino)hexanoyl)amino]hexanoate (SIAXX), succinimidyl-4-(((iodoacetyl)amino)methyl)cyclohexane-1-carboxylate (SIAC), succinimidyl 6-[(iodoacetyl)amino]hexanoate (SIAX) and p-nitrophenyl iodoacetate (NPIA), N-hydroxysuccinimide, N oxysuccinimide and adipic acid N-hydroxysuccinimide diester (SIDEA) and Bis(sulfosuccinimidyl) suberate (BS3), acryloyl halides (e.g. chloride), ethylene glycol bis[succinimidylsuccinate], bis(sulfosuccinimidyl)tri(ethylene glycol) (BS(PEG)3), bis(sulfosuccinimidyl)tetra(ethylene glycol) (BS(PEG)4), bis(sulfosuccinimidyl)penta(ethylene glycol) (BS(PEG)5) and bis(sulfosuccinimidyl)exa(ethylene glycol) (BS(PEG)6), adipic acid dihydrazide (ADH), β-propionamido, nitrophenyl-ethylamine, haloacyl halides, and 6-aminocaproic acid.

7. The immunogenic conjugate according to the preceding claims, wherein said nOMV is obtained by a detergent free process, being released into the fermentation broth and purified using a centrifugation and subsequent filtration; or being released into the fermentation broth and purified using two consecutive Tangential Flow Filtration (TFF) steps.

8. The immunogenic conjugate according to claims 1-7, wherein said nOMV is a GMMA vesicle.

9. A process for preparing the immunogenic conjugate according to claims 1-8, comprising the steps of:
iii) reacting at least a nOMV surface protein residue with the first terminal portion of a divalent linker to obtain a nOMV-linker intermediate; and
iv) reacting said nOMV-linker intermediate with at least one antigen via the second terminal portion of the divalent linker, thus obtaining the nOMV-linker-antigen conjugate.

10. The nOMV-linker intermediate obtained (or obtainable) by the step i) of claim 9.

11. Use of the nOMV-linker intermediate of claim 10 for the preparation of nOMV-linker-antigen according to claims 1-8.

12. An immunogenic composition comprising a conjugate according to any one of claims 1-10 and at least one pharmaceutically acceptable carrier or excipient.

13. The immunogenic conjugate or composition according to any one of claims 1-8 or 12, for use as medicament.

14. The immunogenic conjugate or composition for use according to claim 13 to induce an immune response in a vertebrate.

15. A vaccine comprising the immunogenic conjugates defined in any one of claims 1-8 or the immunogenic composition defined in claim 12.


SEQUENCE LISTING

<110>  GlaxoSmithKline Biologicals SA

<120>  nOMV-ANTIGEN CONJUGATES AND USE THEREOF

<130>  VB66223D1 EP

<150> GB 1619949.9
<151> 2016-11-25

<150> GB 1712101.3
<151> 2017-07-27

<150> EP 17816489.3
<151> 2017-11-23

<160>  16

<170>  PatentIn version 3.5

<210>  1
<211>  247
<212>  PRT
<213>  Neisseria meningitidis

<400>  1

Val Ala Ala Asp Ile Gly Ala Gly Leu Ala Asp Ala Leu Thr Ala Pro
1               5                   10                  15

Leu Asp His Lys Asp Lys Ser Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Ile Tyr Lys Gln Asp His
                85                  90                  95

Ser Ala Val Val Ala Leu Gln Ile Glu Lys Ile Asn Asn Pro Asp Lys
            100                 105                 110

Ile Asp Ser Leu Ile Asn Gln Arg Ser Phe Arg Val Ser Gly Leu Gly
        115                 120                 125

Gly Glu His Thr Ala Phe Asn Gln Leu Pro Asp Gly Lys Ala Glu Tyr
    130                 135                 140

His Gly Lys Ala Phe Ser Ser Asp Asp Ala Gly Gly Lys Leu Thr Tyr
145                 150                 155                 160

Thr Ile Asp Phe Ala Ala Lys Gln Gly His Gly Lys Ile Glu His Leu
                165                 170                 175

Lys Thr Pro Glu Gln Asn Val Glu Leu Ala Ala Ala Glu Leu Lys Ala
            180                 185                 190

Asp Glu Lys Ser His Ala Val Ile Leu Gly Asp Thr Arg Tyr Gly Ser
        195                 200                 205

Glu Glu Lys Gly Thr Tyr His Leu Ala Leu Phe Gly Asp Arg Ala Gln
    210                 215                 220

Glu Ile Ala Gly Ser Ala Thr Val Lys Ile Gly Glu Lys Val His Glu
225                 230                 235                 240

Ile Gly Ile Ala Gly Lys Gln
                245

<210>  2
<211>  488
<212>  PRT
<213>  Neisseria meningitidis

<400>  2

Met Phe Lys Arg Ser Val Ile Ala Met Ala Cys Ile Phe Ala Leu Ser
1               5                   10                  15

Ala Cys Gly Gly Gly Gly Gly Gly Ser Pro Asp Val Lys Ser Ala Asp
            20                  25                  30

Thr Leu Ser Lys Pro Ala Ala Pro Val Val Ser Glu Lys Glu Thr Glu
        35                  40                  45

Ala Lys Glu Asp Ala Pro Gln Ala Gly Ser Gln Gly Gln Gly Ala Pro
    50                  55                  60

Ser Ala Gln Gly Ser Gln Asp Met Ala Ala Val Ser Glu Glu Asn Thr
65                  70                  75                  80

Gly Asn Gly Gly Ala Val Thr Ala Asp Asn Pro Lys Asn Glu Asp Glu
                85                  90                  95

Val Ala Gln Asn Asp Met Pro Gln Asn Ala Ala Gly Thr Asp Ser Ser
            100                 105                 110

Thr Pro Asn His Thr Pro Asp Pro Asn Met Leu Ala Gly Asn Met Glu
        115                 120                 125

Asn Gln Ala Thr Asp Ala Gly Glu Ser Ser Gln Pro Ala Asn Gln Pro
    130                 135                 140

Asp Met Ala Asn Ala Ala Asp Gly Met Gln Gly Asp Asp Pro Ser Ala
145                 150                 155                 160

Gly Gly Gln Asn Ala Gly Asn Thr Ala Ala Gln Gly Ala Asn Gln Ala
                165                 170                 175

Gly Asn Asn Gln Ala Ala Gly Ser Ser Asp Pro Ile Pro Ala Ser Asn
            180                 185                 190

Pro Ala Pro Ala Asn Gly Gly Ser Asn Phe Gly Arg Val Asp Leu Ala
        195                 200                 205

Asn Gly Val Leu Ile Asp Gly Pro Ser Gln Asn Ile Thr Leu Thr His
    210                 215                 220

Cys Lys Gly Asp Ser Cys Ser Gly Asn Asn Phe Leu Asp Glu Glu Val
225                 230                 235                 240

Gln Leu Lys Ser Glu Phe Glu Lys Leu Ser Asp Ala Asp Lys Ile Ser
                245                 250                 255

Asn Tyr Lys Lys Asp Gly Lys Asn Asp Lys Phe Val Gly Leu Val Ala
            260                 265                 270

Asp Ser Val Gln Met Lys Gly Ile Asn Gln Tyr Ile Ile Phe Tyr Lys
        275                 280                 285

Pro Lys Pro Thr Ser Phe Ala Arg Phe Arg Arg Ser Ala Arg Ser Arg
    290                 295                 300

Arg Ser Leu Pro Ala Glu Met Pro Leu Ile Pro Val Asn Gln Ala Asp
305                 310                 315                 320

Thr Leu Ile Val Asp Gly Glu Ala Val Ser Leu Thr Gly His Ser Gly
                325                 330                 335

Asn Ile Phe Ala Pro Glu Gly Asn Tyr Arg Tyr Leu Thr Tyr Gly Ala
            340                 345                 350

Glu Lys Leu Pro Gly Gly Ser Tyr Ala Leu Arg Val Gln Gly Glu Pro
        355                 360                 365

Ala Lys Gly Glu Met Leu Ala Gly Ala Ala Val Tyr Asn Gly Glu Val
    370                 375                 380

Leu His Phe His Thr Glu Asn Gly Arg Pro Tyr Pro Thr Arg Gly Arg
385                 390                 395                 400

Phe Ala Ala Lys Val Asp Phe Gly Ser Lys Ser Val Asp Gly Ile Ile
                405                 410                 415

Asp Ser Gly Asp Asp Leu His Met Gly Thr Gln Lys Phe Lys Ala Ala
            420                 425                 430

Ile Asp Gly Asn Gly Phe Lys Gly Thr Trp Thr Glu Asn Gly Ser Gly
        435                 440                 445

Asp Val Ser Gly Lys Phe Tyr Gly Pro Ala Gly Glu Glu Val Ala Gly
    450                 455                 460

Lys Tyr Ser Tyr Arg Pro Thr Asp Ala Glu Lys Gly Gly Phe Gly Val
465                 470                 475                 480

Phe Ala Gly Lys Lys Glu Gln Asp
                485

<210>  3
<211>  362
<212>  PRT
<213>  Neisseria meningitidis

<400>  3

Met Lys His Phe Pro Ser Lys Val Leu Thr Thr Ala Ile Leu Ala Thr
1               5                   10                  15

Phe Cys Ser Gly Ala Leu Ala Ala Thr Ser Asp Asp Asp Val Lys Lys
            20                  25                  30

Ala Ala Thr Val Ala Ile Val Ala Ala Tyr Asn Asn Gly Gln Glu Ile
        35                  40                  45

Asn Gly Phe Lys Ala Gly Glu Thr Ile Tyr Asp Ile Gly Glu Asp Gly
    50                  55                  60

Thr Ile Thr Gln Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
            100                 105                 110

Ser Glu Ile Glu Lys Leu Thr Thr Lys Leu Ala Asp Thr Asp Ala Ala
        115                 120                 125

Leu Ala Asp Thr Asp Ala Ala Leu Asp Glu Thr Thr Asn Ala Leu Asn
    130                 135                 140

Lys Leu Gly Glu Asn Ile Thr Thr Phe Ala Glu Glu Thr Lys Thr Asn
145                 150                 155                 160

Ile Val Lys Ile Asp Glu Lys Leu Glu Ala Val Ala Asp Thr Val Asp
                165                 170                 175

Lys His Ala Glu Ala Phe Asn Asp Ile Ala Asp Ser Leu Asp Glu Thr
            180                 185                 190

Asn Thr Lys Ala Asp Glu Ala Val Lys Thr Ala Asn Glu Ala Lys Gln
        195                 200                 205

Thr Ala Glu Glu Thr Lys Gln Asn Val Asp Ala Lys Val Lys Ala Ala
    210                 215                 220

Glu Thr Ala Ala Gly Lys Ala Glu Ala Ala Ala Gly Thr Ala Asn Thr
225                 230                 235                 240

Ala Ala Asp Lys Ala Glu Ala Val Ala Ala Lys Val Thr Asp Ile Lys
                245                 250                 255

Ala Asp Ile Ala Thr Asn Lys Ala Asp Ile Ala Lys Asn Ser Ala Arg
            260                 265                 270

Ile Asp Ser Leu Asp Lys Asn Val Ala Asn Leu Arg Lys Glu Thr Arg
        275                 280                 285

Gln Gly Leu Ala Glu Gln Ala Ala Leu Ser Gly Leu Phe Gln Pro Tyr
    290                 295                 300

Asn Val Gly Arg Phe Asn Val Thr Ala Ala Val Gly Gly Tyr Lys Ser
305                 310                 315                 320

Glu Ser Ala Val Ala Ile Gly Thr Gly Phe Arg Phe Thr Glu Asn Phe
                325                 330                 335

Ala Ala Lys Ala Gly Val Ala Val Gly Thr Ser Ser Gly Ser Ser Ala

340                 345                 350

Ala Tyr His Val Gly Val Asn Tyr Glu Trp
        355                 360

<210>  4
<211>  174
<212>  PRT
<213>  Neisseria meningitidis

<400>  4

Met Lys Lys Ala Leu Ala Thr Leu Ile Ala Leu Ala Leu Pro Ala Ala
1               5                   10                  15

Ala Leu Ala Glu Gly Ala Ser Gly Phe Tyr Val Gln Ala Asp Ala Ala
            20                  25                  30

His Ala Lys Ala Ser Ser Ser Leu Gly Ser Ala Lys Gly Phe Ser Pro
        35                  40                  45

Arg Ile Ser Ala Gly Tyr Arg Ile Asn Asp Leu Arg Phe Ala Val Asp
    50                  55                  60

Tyr Thr Arg Tyr Lys Asn Tyr Lys Ala Pro Ser Thr Asp Phe Lys Leu
65                  70                  75                  80

Tyr Ser Ile Gly Ala Ser Ala Ile Tyr Asp Phe Asp Thr Gln Ser Pro
                85                  90                  95

Val Lys Pro Tyr Leu Gly Ala Arg Leu Ser Leu Asn Arg Ala Ser Val
            100                 105                 110

Asp Leu Gly Gly Ser Asp Ser Phe Ser Gln Thr Ser Ile Gly Leu Gly
        115                 120                 125

Val Leu Thr Gly Val Ser Tyr Ala Val Thr Pro Asn Val Asp Leu Asp
    130                 135                 140

Ala Gly Tyr Arg Tyr Asn Tyr Ile Gly Lys Val Asn Thr Val Lys Asn
145                 150                 155                 160

Val Arg Ser Gly Glu Leu Ser Ala Gly Val Arg Val Lys Phe
                165                 170

<210>  5
<211>  591
<212>  PRT
<213>  Neisseria meningitidis

<400> 5

Met Asn Lys Ile Tyr Arg Ile Ile Trp Asn Ser Ala Leu Asn Ala Trp
1               5                   10                  15

Val Val Val Ser Glu Leu Thr Arg Asn His Thr Lys Arg Ala Ser Ala
            20                  25                  30

Thr Val Lys Thr Ala Val Leu Ala Thr Leu Leu Phe Ala Thr Val Gln
        35                  40                  45

Ala Ser Ala Asn Asn Glu Glu Gln Glu Glu Asp Leu Tyr Leu Asp Pro
    50                  55                  60

Val Gln Arg Thr Val Ala Val Leu Ile Val Asn Ser Asp Lys Glu Gly
65                  70                  75                  80

Thr Gly Glu Lys Glu Lys Val Glu Glu Asn Ser Asp Trp Ala Val Tyr
                85                  90                  95

Phe Asn Glu Lys Gly Val Leu Thr Ala Arg Glu Ile Thr Leu Lys Ala
            100                 105                 110

Gly Asp Asn Leu Lys Ile Lys Gln Asn Gly Thr Asn Phe Thr Tyr Ser
        115                 120                 125

Leu Lys Lys Asp Leu Thr Asp Leu Thr Ser Val Gly Thr Glu Lys Leu
    130                 135                 140

Ser Phe Ser Ala Asn Gly Asn Lys Val Asn Ile Thr Ser Asp Thr Lys
145                 150                 155                 160

Gly Leu Asn Phe Ala Lys Glu Thr Ala Gly Thr Asn Gly Asp Thr Thr
                165                 170                 175

Val His Leu Asn Gly Ile Gly Ser Thr Leu Thr Asp Thr Leu Leu Asn
            180                 185                 190

Thr Gly Ala Thr Thr Asn Val Thr Asn Asp Asn Val Thr Asp Asp Glu
        195                 200                 205

Lys Lys Arg Ala Ala Ser Val Lys Asp Val Leu Asn Ala Gly Trp Asn
    210                 215                 220

Ile Lys Gly Val Lys Pro Gly Thr Thr Ala Ser Asp Asn Val Asp Phe
225                 230                 235                 240

Val Arg Thr Tyr Asp Thr Val Glu Phe Leu Ser Ala Asp Thr Lys Thr

245                 250                 255

Thr Thr Val Asn Val Glu Ser Lys Asp Asn Gly Lys Lys Thr Glu Val
            260                 265                 270

Lys Ile Gly Ala Lys Thr Ser Val Ile Lys Glu Lys Asp Gly Lys Leu
        275                 280                 285

Val Thr Gly Lys Asp Lys Gly Glu Asn Gly Ser Ser Thr Asp Glu Gly
    290                 295                 300

Glu Gly Leu Val Thr Ala Lys Glu Val Ile Asp Ala Val Asn Lys Ala
305                 310                 315                 320

Gly Trp Arg Met Lys Thr Thr Thr Ala Asn Gly Gln Thr Gly Gln Ala
                325                 330                 335

Asp Lys Phe Glu Thr Val Thr Ser Gly Thr Asn Val Thr Phe Ala Ser
            340                 345                 350

Gly Lys Gly Thr Thr Ala Thr Val Ser Lys Asp Asp Gln Gly Asn Ile
        355                 360                 365

Thr Val Met Tyr Asp Val Asn Val Gly Asp Ala Leu Asn Val Asn Gln
    370                 375                 380

Leu Gln Asn Ser Gly Trp Asn Leu Asp Ser Lys Ala Val Ala Gly Ser
385                 390                 395                 400

Ser Gly Lys Val Ile Ser Gly Asn Val Ser Pro Ser Lys Gly Lys Met
                405                 410                 415

Asp Glu Thr Val Asn Ile Asn Ala Gly Asn Asn Ile Glu Ile Thr Arg
            420                 425                 430

Asn Gly Lys Asn Ile Asp Ile Ala Thr Ser Met Thr Pro Gln Phe Ser
        435                 440                 445

Ser Val Ser Leu Gly Ala Gly Ala Asp Ala Pro Thr Leu Ser Val Asp
    450                 455                 460

Gly Asp Ala Leu Asn Val Gly Ser Lys Lys Asp Asn Lys Pro Val Arg
465                 470                 475                 480

Ile Thr Asn Val Ala Pro Gly Val Lys Glu Gly Asp Val Thr Asn Val
                485                 490                 495

Ala Gln Leu Lys Gly Val Ala Gln Asn Leu Asn Asn Arg Ile Asp Asn
            500                 505                 510

Val Asp Gly Asn Ala Arg Ala Gly Ile Ala Gln Ala Ile Ala Thr Ala
        515                 520                 525

Gly Leu Val Gln Ala Tyr Leu Pro Gly Lys Ser Met Met Ala Ile Gly
    530                 535                 540

Gly Gly Thr Tyr Arg Gly Glu Ala Gly Tyr Ala Ile Gly Tyr Ser Ser
545                 550                 555                 560

Ile Ser Asp Gly Gly Asn Trp Ile Ile Lys Gly Thr Ala Ser Gly Asn
                565                 570                 575

Ser Arg Gly His Phe Gly Ala Ser Ala Ser Val Gly Tyr Gln Trp
            580                 585                 590

<210> 6
<211> 1457
<212> PRT
<213> Neisseria meningitidis

<400> 6

Met Lys Thr Thr Asp Lys Arg Thr Thr Glu Thr His Arg Lys Ala Pro
1               5                   10                  15

Lys Thr Gly Arg Ile Arg Phe Ser Pro Ala Tyr Leu Ala Ile Cys Leu
            20                  25                  30

Ser Phe Gly Ile Leu Pro Gln Ala Trp Ala Gly His Thr Tyr Phe Gly
        35                  40                  45

Ile Asn Tyr Gln Tyr Tyr Arg Asp Phe Ala Glu Asn Lys Gly Lys Phe
    50                  55                  60

Ala Val Gly Ala Lys Asp Ile Glu Val Tyr Asn Lys Lys Gly Glu Leu
65                  70                  75                  80

Val Gly Lys Ser Met Thr Lys Ala Pro Met Ile Asp Phe Ser Val Val
                85                  90                  95

Ser Arg Asn Gly Val Ala Ala Leu Val Gly Asp Gln Tyr Ile Val Ser
            100                 105                 110

Val Ala His Asn Gly Gly Tyr Asn Asn Val Asp Phe Gly Ala Glu Gly
        115                 120                 125

Arg Asn Pro Asp Gln His Arg Phe Thr Tyr Lys Ile Val Lys Arg Asn
    130                 135                 140

Asn Tyr Lys Ala Gly Thr Lys Gly His Pro Tyr Gly Gly Asp Tyr His
145                 150                 155                 160

Met Pro Arg Leu His Lys Phe Val Thr Asp Ala Glu Pro Val Glu Met
                165                 170                 175

Thr Ser Tyr Met Asp Gly Arg Lys Tyr Ile Asp Gln Asn Asn Tyr Pro
            180                 185                 190

Asp Arg Val Arg Ile Gly Ala Gly Arg Gln Tyr Trp Arg Ser Asp Glu
        195                 200                 205

Asp Glu Pro Asn Asn Arg Glu Ser Ser Tyr His Ile Ala Ser Ala Tyr
    210                 215                 220

Ser Trp Leu Val Gly Gly Asn Thr Phe Ala Gln Asn Gly Ser Gly Gly
225                 230                 235                 240

Gly Thr Val Asn Leu Gly Ser Glu Lys Ile Lys His Ser Pro Tyr Gly
                245                 250                 255

Phe Leu Pro Thr Gly Gly Ser Phe Gly Asp Ser Gly Ser Pro Met Phe
            260                 265                 270

Ile Tyr Asp Ala Gln Lys Gln Lys Trp Leu Ile Asn Gly Val Leu Gln
        275                 280                 285

Thr Gly Asn Pro Tyr Ile Gly Lys Ser Asn Gly Phe Gln Leu Val Arg
    290                 295                 300

Lys Asp Trp Phe Tyr Asp Glu Ile Phe Ala Gly Asp Thr His Ser Val
305                 310                 315                 320

Phe Tyr Glu Pro Arg Gln Asn Gly Lys Tyr Ser Phe Asn Asp Asp Asn
                325                 330                 335

Asn Gly Thr Gly Lys Ile Asn Ala Lys His Glu His Asn Ser Leu Pro
            340                 345                 350

Asn Arg Leu Lys Thr Arg Thr Val Gln Leu Phe Asn Val Ser Leu Ser
        355                 360                 365

Glu Thr Ala Arg Glu Pro Val Tyr His Ala Ala Gly Gly Val Asn Ser
    370                 375                 380

Tyr Arg Pro Arg Leu Asn Asn Gly Glu Asn Ile Ser Phe Ile Asp Glu
385                 390                 395                 400

Gly Lys Gly Glu Leu Ile Leu Thr Ser Asn Ile Asn Gln Gly Ala Gly
                405                 410                 415

Gly Leu Tyr Phe Gln Gly Asp Phe Thr Val Ser Pro Glu Asn Asn Glu
            420                 425                 430

Thr Trp Gln Gly Ala Gly Val His Ile Ser Glu Asp Ser Thr Val Thr
        435                 440                 445

Trp Lys Val Asn Gly Val Ala Asn Asp Arg Leu Ser Lys Ile Gly Lys
    450                 455                 460

Gly Thr Leu His Val Gln Ala Lys Gly Glu Asn Gln Gly Ser Ile Ser
465                 470                 475                 480

Val Gly Asp Gly Thr Val Ile Leu Asp Gln Gln Ala Asp Asp Lys Gly
                485                 490                 495

Lys Lys Gln Ala Phe Ser Glu Ile Gly Leu Val Ser Gly Arg Gly Thr
            500                 505                 510

Val Gln Leu Asn Ala Asp Asn Gln Phe Asn Pro Asp Lys Leu Tyr Phe
        515                 520                 525

Gly Phe Arg Gly Gly Arg Leu Asp Leu Asn Gly His Ser Leu Ser Phe
    530                 535                 540

His Arg Ile Gln Asn Thr Asp Glu Gly Ala Met Ile Val Asn His Asn
545                 550                 555                 560

Gln Asp Lys Glu Ser Thr Val Thr Ile Thr Gly Asn Lys Asp Ile Ala
                565                 570                 575

Thr Thr Gly Asn Asn Asn Ser Leu Asp Ser Lys Lys Glu Ile Ala Tyr
            580                 585                 590

Asn Gly Trp Phe Gly Glu Lys Asp Thr Thr Lys Thr Asn Gly Arg Leu
        595                 600                 605

Asn Leu Val Tyr Gln Pro Ala Ala Glu Asp Arg Thr Leu Leu Leu Ser
    610                 615                 620

Gly Gly Thr Asn Leu Asn Gly Asn Ile Thr Gln Thr Asn Gly Lys Leu
625                 630                 635                 640

Phe Phe Ser Gly Arg Pro Thr Pro His Ala Tyr Asn His Leu Asn Asp
                645                 650                 655

His Trp Ser Gln Lys Glu Gly Ile Pro Arg Gly Glu Ile Val Trp Asp
            660                 665                 670

Asn Asp Trp Ile Asn Arg Thr Phe Lys Ala Glu Asn Phe Gln Ile Lys
        675                 680                 685

Gly Gly Gln Ala Val Val Ser Arg Asn Val Ala Lys Val Lys Gly Asp
    690                 695                 700

Trp His Leu Ser Asn His Ala Gln Ala Val Phe Gly Val Ala Pro His
705                 710                 715                 720

Gln Ser His Thr Ile Cys Thr Arg Ser Asp Trp Thr Gly Leu Thr Asn
                725                 730                 735

Cys Val Glu Lys Thr Ile Thr Asp Asp Lys Val Ile Ala Ser Leu Thr
            740                 745                 750

Lys Thr Asp Ile Ser Gly Asn Val Asp Leu Ala Asp His Ala His Leu
        755                 760                 765

Asn Leu Thr Gly Leu Ala Thr Leu Asn Gly Asn Leu Ser Ala Asn Gly
    770                 775                 780

Asp Thr Arg Tyr Thr Val Ser His Asn Ala Thr Gln Asn Gly Asn Leu
785                 790                 795                 800

Ser Leu Val Gly Asn Ala Gln Ala Thr Phe Asn Gln Ala Thr Leu Asn
                805                 810                 815

Gly Asn Thr Ser Ala Ser Gly Asn Ala Ser Phe Asn Leu Ser Asp His
            820                 825                 830

Ala Val Gln Asn Gly Ser Leu Thr Leu Ser Gly Asn Ala Lys Ala Asn
        835                 840                 845

Val Ser His Ser Ala Leu Asn Gly Asn Val Ser Leu Ala Asp Lys Ala
    850                 855                 860

Val Phe His Phe Glu Ser Ser Arg Phe Thr Gly Gln Ile Ser Gly Gly
865                 870                 875                 880

Lys Asp Thr Ala Leu His Leu Lys Asp Ser Glu Trp Thr Leu Pro Ser

                885                 890                 895

Gly Thr Glu Leu Gly Asn Leu Asn Leu Asp Asn Ala Thr Ile Thr Leu
            900                 905                 910

Asn Ser Ala Tyr Arg His Asp Ala Ala Gly Ala Gln Thr Gly Ser Ala
        915                 920                 925

Thr Asp Ala Pro Arg Arg Arg Ser Arg Arg Ser Arg Arg Ser Leu Leu
    930                 935                 940

Ser Val Thr Pro Pro Thr Ser Val Glu Ser Arg Phe Asn Thr Leu Thr
945                 950                 955                 960

Val Asn Gly Lys Leu Asn Gly Gln Gly Thr Phe Arg Phe Met Ser Glu
                965                 970                 975

Leu Phe Gly Tyr Arg Ser Asp Lys Leu Lys Leu Ala Glu Ser Ser Glu
            980                 985                 990

Gly Thr Tyr Thr Leu Ala Val Asn Asn Thr Gly Asn Glu Pro Ala Ser
        995                 1000                1005

Leu Glu Gln Leu Thr Val Val Glu Gly Lys Asp Asn Lys Pro Leu
    1010                1015                1020

Ser Glu Asn Leu Asn Phe Thr Leu Gln Asn Glu His Val Asp Ala
    1025                1030                1035

Gly Ala Trp Arg Tyr Gln Leu Ile Arg Lys Asp Gly Glu Phe Arg
    1040                1045                1050

Leu His Asn Pro Val Lys Glu Gln Glu Leu Ser Asp Lys Leu Gly
    1055                1060                1065

Lys Ala Glu Ala Lys Lys Gln Ala Glu Lys Asp Asn Ala Gln Ser
    1070                1075                1080

Leu Asp Ala Leu Ile Ala Ala Gly Arg Asp Ala Val Glu Lys Thr
    1085                1090                1095

Glu Ser Val Ala Glu Pro Ala Arg Gln Ala Gly Gly Glu Asn Val
    1100                1105                1110

Gly Ile Met Gln Ala Glu Glu Glu Lys Lys Arg Val Gln Ala Asp
    1115                1120                1125

Lys Asp Thr Ala Leu Ala Lys Gln Arg Glu Ala Glu Thr Arg Pro
    1130                1135                1140

Ala Thr Thr Ala Phe Pro Arg Ala Arg Arg Ala Arg Arg Asp Leu
    1145                1150                1155

Pro Gln Leu Gln Pro Gln Pro Gln Pro Gln Pro Gln Arg Asp Leu
    1160                1165                1170

Ile Ser Arg Tyr Ala Asn Ser Gly Leu Ser Glu Phe Ser Ala Thr
    1175                1180                1185

Leu Asn Ser Val Phe Ala Val Gln Asp Glu Leu Asp Arg Val Phe
    1190                1195                1200

Ala Glu Asp Arg Arg Asn Ala Val Trp Thr Ser Gly Ile Arg Asp
    1205                1210                1215

Thr Lys His Tyr Arg Ser Gln Asp Phe Arg Ala Tyr Arg Gln Gln
    1220                1225                1230

Thr Asp Leu Arg Gln Ile Gly Met Gln Lys Asn Leu Gly Ser Gly
    1235                1240                1245

Arg Val Gly Ile Leu Phe Ser His Asn Arg Thr Glu Asn Thr Phe
    1250                1255                1260

Asp Asp Gly Ile Gly Asn Ser Ala Arg Leu Ala His Gly Ala Val
    1265                1270                1275

Phe Gly Gln Tyr Gly Ile Asp Arg Phe Tyr Ile Gly Ile Ser Ala
    1280                1285                1290

Gly Ala Gly Phe Ser Ser Gly Ser Leu Ser Asp Gly Ile Gly Gly
    1295                1300                1305

Lys Ile Arg Arg Arg Val Leu His Tyr Gly Ile Gln Ala Arg Tyr
    1310                1315                1320

Arg Ala Gly Phe Gly Gly Phe Gly Ile Glu Pro His Ile Gly Ala
    1325                1330                1335

Thr Arg Tyr Phe Val Gln Lys Ala Asp Tyr Arg Tyr Glu Asn Val
    1340                1345                1350

Asn Ile Ala Thr Pro Gly Leu Ala Phe Asn Arg Tyr Arg Ala Gly
    1355                1360                1365

Ile Lys Ala Asp Tyr Ser Phe Lys Pro Ala Gln His Ile Ser Ile
    1370                1375                1380

Thr Pro Tyr Leu Ser Leu Ser Tyr Thr Asp Ala Ala Ser Gly Lys
    1385                1390                1395

Val Arg Thr Arg Val Asn Thr Ala Val Leu Ala Gln Asp Phe Gly
    1400                1405                1410

Lys Thr Arg Ser Ala Glu Trp Gly Val Asn Ala Glu Ile Lys Gly
    1415                1420                1425

Phe Thr Leu Ser Leu His Ala Ala Ala Ala Lys Gly Pro Gln Leu
    1430                1435                1440

Glu Ala Gln His Ser Ala Gly Ile Lys Leu Gly Tyr Arg Trp
    1445                1450                1455

<210> 7
<211> 327
<212> PRT
<213> Neisseria meningitidis

<400> 7

Ala Thr Asn Asp Asp Asp Val Lys Lys Ala Ala Thr Val Ala Ile Ala
1               5                   10                  15

Ala Ala Tyr Asn Asn Gly Gln Glu Ile Asn Gly Phe Lys Ala Gly Glu
            20                  25                  30

Thr Ile Tyr Asp Ile Asp Glu Asp Gly Thr Ile Thr Lys Lys Asp Ala
        35                  40                  45

Thr Ala Ala Asp Val Glu Ala Asp Asp Phe Lys Gly Leu Gly Leu Lys
    50                  55                  60

Lys Val Val Thr Asn Leu Thr Lys Thr Val Asn Glu Asn Lys Gln Asn
65                  70                  75                  80

Val Asp Ala Lys Val Lys Ala Ala Glu Ser Glu Ile Glu Lys Leu Thr
                85                  90                  95

Thr Lys Leu Ala Asp Thr Asp Ala Ala Leu Ala Asp Thr Asp Ala Ala
            100                 105                 110

Leu Asp Ala Thr Thr Asn Ala Leu Asn Lys Leu Gly Glu Asn Ile Thr
        115                 120                 125

Thr Phe Ala Glu Glu Thr Lys Thr Asn Ile Val Lys Ile Asp Glu Lys
    130                 135                 140

Leu Glu Ala Val Ala Asp Thr Val Asp Lys His Ala Glu Ala Phe Asn
145                 150                 155                 160

Asp Ile Ala Asp Ser Leu Asp Glu Thr Asn Thr Lys Ala Asp Glu Ala
                165                 170                 175

Val Lys Thr Ala Asn Glu Ala Lys Gln Thr Ala Glu Glu Thr Lys Gln
            180                 185                 190

Asn Val Asp Ala Lys Val Lys Ala Ala Glu Thr Ala Ala Gly Lys Ala
        195                 200                 205

Glu Ala Ala Ala Gly Thr Ala Asn Thr Ala Ala Asp Lys Ala Glu Ala
    210                 215                 220

Val Ala Ala Lys Val Thr Asp Ile Lys Ala Asp Ile Ala Thr Asn Lys
225                 230                 235                 240

Asp Asn Ile Ala Lys Lys Ala Asn Ser Ala Asp Val Tyr Thr Arg Glu
                245                 250                 255

Glu Ser Asp Ser Lys Phe Val Arg Ile Asp Gly Leu Asn Ala Thr Thr
            260                 265                 270

Glu Lys Leu Asp Thr Arg Leu Ala Ser Ala Glu Lys Ser Ile Ala Asp
        275                 280                 285

His Asp Thr Arg Leu Asn Gly Leu Asp Lys Thr Val Ser Asp Leu Arg
    290                 295                 300

Lys Glu Thr Arg Gln Gly Leu Ala Glu Gln Ala Ala Leu Ser Gly Leu
305                 310                 315                 320

Phe Gln Pro Tyr Asn Val Gly
                325

<210>  8
<211>  248
<212>  PRT
<213>  Neisseria meningitidis

<400>  8

Val Ala Ala Asp Ile Gly Ala Gly Leu Ala Asp Ala Leu Thr Ala Pro
1               5                   10                  15

Leu Asp His Lys Asp Lys Gly Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Val Tyr Lys Gln Ser His
                85                  90                  95

Ser Ala Leu Thr Ala Phe Gln Thr Glu Gln Ile Gln Asp Ser Glu His
            100                 105                 110

Ser Gly Lys Met Val Ala Lys Arg Gln Phe Arg Ile Gly Asp Ile Ala
        115                 120                 125

Gly Glu His Thr Ser Phe Asp Lys Leu Pro Glu Gly Gly Arg Ala Thr
    130                 135                 140

Tyr Arg Gly Thr Ala Phe Gly Ser Asp Asp Ala Gly Gly Lys Leu Thr
145                 150                 155                 160

Tyr Thr Ile Asp Phe Ala Ala Lys Gln Gly Asn Gly Lys Ile Glu His
                165                 170                 175

Leu Lys Ser Pro Glu Leu Asn Val Asp Leu Ala Ala Ala Asp Ile Lys
            180                 185                 190

Pro Asp Gly Lys Arg His Ala Val Ile Ser Gly Ser Val Leu Tyr Asn
        195                 200                 205

Gln Ala Glu Lys Gly Ser Tyr Ser Leu Gly Ile Phe Gly Gly Lys Ala
    210                 215                 220

Gln Glu Val Ala Gly Ser Ala Glu Val Lys Thr Val Asn Gly Ile Arg
225                 230                 235                 240

His Ile Gly Leu Ala Ala Lys Gln
                245

<210>  9
<211>  250

<212>  PRT
<213>  Neisseria meningitidis

<400>  9

Val Ala Ala Asp Ile Gly Thr Gly Leu Ala Asp Ala Leu Thr Ala Pro
1               5                   10                  15

Leu Asp His Lys Asp Lys Gly Leu Lys Ser Leu Thr Leu Glu Asp Ser
            20                  25                  30

Ile Pro Gln Asn Gly Thr Leu Thr Leu Ser Ala Gln Gly Ala Glu Lys
        35                  40                  45

Thr Phe Lys Ala Gly Asp Lys Asp Asn Ser Leu Asn Thr Gly Lys Leu
    50                  55                  60

Lys Asn Asp Lys Ile Ser Arg Phe Asp Phe Val Gln Lys Ile Glu Val
65                  70                  75                  80

Asp Gly Gln Thr Ile Thr Leu Ala Ser Gly Glu Phe Gln Ile Tyr Lys
                85                  90                  95

Gln Asn His Ser Ala Val Val Ala Leu Gln Ile Glu Lys Ile Asn Asn
            100                 105                 110

Pro Asp Lys Thr Asp Ser Leu Ile Asn Gln Arg Ser Phe Leu Val Ser
        115                 120                 125

Gly Leu Gly Gly Glu His Thr Ala Phe Asn Gln Leu Pro Gly Gly Lys
    130                 135                 140

Ala Glu Tyr His Gly Lys Ala Phe Ser Ser Asp Asp Pro Asn Gly Arg
145                 150                 155                 160

Leu His Tyr Ser Ile Asp Phe Thr Lys Lys Gln Gly Tyr Gly Arg Ile
                165                 170                 175

Glu His Leu Lys Thr Leu Glu Gln Asn Val Glu Leu Ala Ala Ala Glu
            180                 185                 190

Leu Lys Ala Asp Glu Lys Ser His Ala Val Ile Leu Gly Asp Thr Arg
        195                 200                 205

Tyr Gly Ser Glu Glu Lys Gly Thr Tyr His Leu Ala Leu Phe Gly Asp
    210                 215                 220

Arg Ala Gln Glu Ile Ala Gly Ser Ala Thr Val Lys Ile Gly Glu Lys
225                 230                 235                 240

Val His Glu Ile Gly Ile Ala Gly Lys Gln
                245                 250

<210>  10
<211>  171
<212>  PRT
<213>  P.falciparum

<400>  10

Lys Val Thr Val Asp Thr Val Cys Lys Arg Gly Phe Leu Ile Gln Met
1               5                   10                  15

Ser Gly His Leu Glu Cys Lys Cys Glu Asn Asp Leu Val Leu Val Asn
            20                  25                  30

Glu Glu Thr Cys Glu Glu Lys Val Leu Lys Cys Asp Glu Lys Thr Val
        35                  40                  45

Asn Lys Pro Cys Gly Asp Phe Ser Lys Cys Ile Lys Ile Asp Gly Asn
    50                  55                  60

Pro Val Ser Tyr Ala Cys Lys Cys Asn Leu Gly Tyr Asp Met Val Asn
65                  70                  75                  80

Asn Val Cys Ile Pro Asn Glu Cys Lys Gln Val Thr Cys Gly Asn Gly
                85                  90                  95

Lys Cys Ile Leu Asp Thr Ser Asn Pro Val Lys Thr Gly Val Cys Ser
            100                 105                 110

Cys Asn Ile Gly Lys Val Pro Asn Val Gln Asp Gln Asn Lys Cys Ser
        115                 120                 125

Lys Asp Gly Glu Thr Lys Cys Ser Leu Lys Cys Leu Lys Glu Gln Glu
    130                 135                 140

Thr Cys Lys Ala Val Asp Gly Ile Tyr Lys Cys Asp Cys Lys Asp Gly
145                 150                 155                 160

Phe Ile Ile Asp Gln Glu Ser Ser Ile Cys Thr
                165                 170

<210>  11
<211>  630
<212>  PRT
<213>  Artificial

<220>

<223>  pfs48/45-GURLP fusion protein

<400>  11

Ala Glu Arg Ser Thr Ser Glu Asn Arg Asn Lys Arg Ile Gly Gly Pro
1               5                   10                  15

Lys Leu Arg Gly Asn Val Thr Ser Asn Ile Lys Phe Pro Ser Asp Asn
            20                  25                  30

Lys Gly Lys Ile Ile Arg Gly Ser Asn Asp Lys Leu Asn Lys Asn Ser
        35                  40                  45

Glu Asp Val Leu Glu Gln Ser Glu Lys Ser Leu Val Ser Glu Asn Val
    50                  55                  60

Pro Ser Gly Leu Asp Ile Asp Asp Ile Pro Lys Glu Ser Ile Phe Ile
65                  70                  75                  80

Gln Glu Asp Gln Glu Gly Gln Thr His Ser Glu Leu Asn Pro Glu Thr
                85                  90                  95

Ser Glu His Ser Lys Asp Leu Asn Asn Asn Gly Ser Lys Asn Glu Ser
            100                 105                 110

Ser Asp Ile Ile Ser Glu Asn Asn Lys Ser Asn Lys Val Gln Asn His
        115                 120                 125

Phe Glu Ser Leu Ser Asp Leu Glu Leu Leu Glu Asn Ser Ser Gln Asp
    130                 135                 140

Asn Leu Asp Lys Asp Thr Ile Ser Thr Glu Pro Phe Pro Asn Gln Lys
145                 150                 155                 160

His Lys Asp Leu Gln Gln Asp Leu Asn Asp Glu Pro Leu Glu Pro Phe
                165                 170                 175

Pro Thr Gln Ile His Lys Asp Tyr Lys Glu Lys Asn Leu Ile Asn Glu
            180                 185                 190

Glu Asp Ser Glu Pro Phe Pro Arg Gln Lys His Lys Lys Val Asp Asn
        195                 200                 205

His Asn Glu Glu Lys Asn Val Phe His Glu Asn Gly Ser Ala Asn Gly
    210                 215                 220

Asn Gln Gly Ser Leu Lys Leu Lys Ser Phe Asp Glu His Leu Lys Asp
225                 230                 235                 240

Glu Lys Ile Glu Asn Glu Pro Leu Val His Glu Asn Leu Ser Ile Pro
                245                 250                 255
Asn Asp Pro Ile Glu Gln Ile Leu Asn Gln Pro Glu Gln Glu Thr Asn
            260                 265                 270
Ile Gln Glu Gln Leu Tyr Asn Glu Lys Gln Asn Val Glu Glu Lys Gln
        275                 280                 285
Asn Ser Gln Ile Pro Ser Leu Asp Leu Lys Glu Pro Thr Asn Glu Asp
    290                 295                 300
Ile Leu Pro Asn His Asn Pro Leu Glu Asn Ile Lys Gln Ser Glu Ser
305                 310                 315                 320
Glu Ile Asn His Val Gln Asp His Ala Leu Pro Lys Glu Asn Ile Ile
                325                 330                 335
Asp Lys Leu Asp Asn Gln Lys Glu His Ile Asp Gln Ser Gln His Asn
            340                 345                 350
Ile Asn Val Leu Gln Glu Asn Asn Ile Asn Asn His Gln Leu Glu Pro
        355                 360                 365
Gln Glu Lys Pro Asn Ile Glu Ser Phe Glu Pro Lys Asn Ile Asp Ser
    370                 375                 380
Glu Ile Ile Leu Pro Glu Asn Val Glu Thr Glu Glu Ile Ile Asp Asp
385                 390                 395                 400
Val Pro Ser Pro Lys His Ser Asn His Glu Thr Phe Glu Glu Glu Thr
                405                 410                 415
Ser Glu Ser Glu His Glu Glu Ala Val Ser Glu Lys Asn Ala His Glu
            420                 425                 430
Thr Val Glu His Glu Glu Thr Val Ser Gln Glu Ser Asn Pro Glu Lys
        435                 440                 445
Ala Asp Asn Asp Gly Asn Val Ser Gln Asn Ser Asn Asn Glu Leu Asn
    450                 455                 460
Glu Asn Glu Phe Val Glu Ser Glu Lys Ser Glu His Glu Ala Arg Ser
465                 470                 475                 480
Lys Pro Lys Tyr Glu Lys Lys Val Ile His Gly Cys Asn Phe Ser Ser
                485                 490                 495

Asn Val Ser Ser Lys His Thr Phe Thr Asp Ser Leu Asp Ile Ser Leu
            500                 505                 510

Val Asp Asp Ser Ala His Ile Ser Cys Asn Val His Leu Ser Glu Pro
        515                 520                 525

Lys Tyr Asn His Leu Val Gly Leu Asn Cys Pro Gly Asp Ile Ile Pro
    530                 535                 540

Asp Cys Phe Phe Gln Val Tyr Gln Pro Glu Ser Glu Glu Leu Glu Pro
545                 550                 555                 560

Ser Asn Ile Val Tyr Leu Asp Ser Gln Ile Asn Ile Gly Asp Ile Glu
                565                 570                 575

Tyr Tyr Glu Asp Ala Glu Gly Asp Asp Lys Ile Lys Leu Phe Gly Ile
            580                 585                 590

Val Gly Ser Ile Pro Lys Thr Thr Ser Phe Thr Cys Ile Cys Lys Lys
        595                 600                 605

Asp Lys Lys Ser Ala Tyr Met Thr Val Thr Ile Asp Ser Ala Arg Ser
    610                 615                 620

His His His His His His
625                 630

<210> 12
<211> 394
<212> PRT
<213> circumsporozoite

<400> 12

Met Leu Phe Gln Glu Tyr Gln Cys Tyr Gly Ser Ser Ser Asn Thr Arg
1               5                   10                  15

Val Leu Asn Glu Leu Asn Tyr Asp Asn Ala Gly Thr Asn Leu Tyr Asn
            20                  25                  30

Glu Leu Glu Met Asn Tyr Tyr Gly Lys Gln Glu Asn Trp Tyr Ser Leu
        35                  40                  45

Lys Lys Asn Ser Arg Ser Leu Gly Glu Asn Asp Asp Gly Asn Asn Asn
    50                  55                  60

Asn Gly Asp Asn Gly Arg Glu Gly Lys Asp Glu Asp Lys Arg Asp Gly
65                  70                  75                  80

Asn Asn Glu Asp Asn Glu Lys Leu Arg Lys Pro Lys His Lys Lys Leu
                85                  90                  95

Lys Gln Pro Gly Asp Gly Asn Pro Asp Pro Asn Ala Asn Pro Asn Val
            100                 105                 110

Asp Pro Asn Ala Asn Pro Asn Val Asp Pro Asn Ala Asn Pro Asn Val
        115                 120                 125

Asp Pro Asn Ala Asn Pro Asn Ala Asn Pro Asn Ala Asn Pro Asn Ala
    130                 135                 140

Asn Pro Asn Ala Asn Pro Asn Ala Asn Pro Asn Ala Asn Pro Asn Ala
145                 150                 155                 160

Asn Pro Asn Ala Asn Pro Asn Ala Asn Pro Asn Ala Asn Pro Asn Ala
                165                 170                 175

Asn Pro Asn Ala Asn Pro Asn Ala Asn Pro Asn Ala Asn Pro Asn Val
            180                 185                 190

Asp Pro Asn Ala Asn Pro Asn Ala Asn Pro Asn Ala Asn Pro Asn Ala
        195                 200                 205

Asn Pro Asn Ala Asn Pro Asn Ala Asn Pro Asn Ala Asn Pro Asn Ala
    210                 215                 220

Asn Pro Asn Ala Asn Pro Asn Ala Asn Pro Asn Ala Asn Pro Asn Ala
225                 230                 235                 240

Asn Pro Asn Ala Asn Pro Asn Ala Asn Pro Asn Ala Asn Pro Asn Ala
                245                 250                 255

Asn Pro Asn Ala Asn Pro Asn Ala Asn Pro Asn Ala Asn Pro Asn Lys
            260                 265                 270

Asn Asn Gln Gly Asn Gly Gln Gly His Asn Met Pro Asn Asp Pro Asn
        275                 280                 285

Arg Asn Val Asp Glu Asn Ala Asn Ala Asn Asn Ala Val Lys Asn Asn
    290                 295                 300

Asn Asn Glu Glu Pro Ser Asp Lys His Ile Glu Lys Tyr Leu Lys Lys
305                 310                 315                 320

Ile Gln Asn Ser Leu Ser Thr Glu Trp Ser Pro Cys Ser Val Thr Cys

325                 330                 335

Gly Asn Gly Ile Gln Val Arg Ile Lys Pro Gly Ser Ala Asn Lys Pro
            340                 345                 350

Lys Asp Glu Leu Asp Tyr Glu Asn Asp Ile Glu Lys Lys Ile Cys Lys
        355                 360                 365

Met Glu Lys Cys Ser Ser Val Phe Asn Val Val Asn Ser Ser Ile Gly
    370                 375                 380

Leu Ile Leu Glu His His His His His His
385                 390

<210>  13
<211>  12
<212>  PRT
<213>  circumsporozoite

<400>  13

Asn Ala Asn Pro Asn Ala Asn Pro Asn Ala Asn Pro
1               5                   10

<210>  14
<211>  134
<212>  PRT
<213>  E. coli

<400>  14

Gln Asp Gln Arg Tyr Ile Ser Ile Arg Asn Thr Asp Thr Ile Trp Leu
1               5                   10                  15

Pro Gly Asn Ile Cys Ala Tyr Gln Phe Arg Leu Asp Asn Gly Gly Asn
            20                  25                  30

Asp Glu Gly Phe Gly Pro Leu Thr Ile Thr Leu Gln Leu Lys Asp Lys
        35                  40                  45

Tyr Gly Gln Thr Leu Val Thr Arg Lys Met Glu Thr Glu Ala Phe Gly
    50                  55                  60

Asp Ser Asn Ala Thr Arg Thr Thr Asp Ala Phe Leu Glu Thr Glu Cys
65                  70                  75                  80

Val Glu Asn Val Ala Thr Thr Glu Ile Ile Lys Ala Thr Glu Glu Ser
                85                  90                  95

Asn Gly His Arg Val Ser Leu Pro Leu Ser Val Phe Asp Pro Gln Asp
            100                 105                 110

Tyr His Pro Leu Leu Ile Thr Val Ser Gly Lys Asn Val Asn Leu Glu
        115                 120                 125

His His His His His His
    130

<210>  15
<211>  1487
<212>  PRT
<213>  E.coli

<400>  15

Asp Thr Pro Ser Val Asp Ser Gly Ser Gly Thr Leu Pro Glu Val Lys
1               5                   10                  15

Pro Asp Pro Thr Pro Thr Pro Glu Pro Thr Pro Glu Pro Thr Pro Asp
            20                  25                  30

Pro Glu Pro Thr Pro Asp Pro Thr Pro Asp Pro Glu Pro Thr Pro Glu
        35                  40                  45

Pro Glu Pro Glu Pro Val Pro Thr Lys Thr Gly Tyr Leu Thr Leu Gly
    50                  55                  60

Gly Ser Gln Arg Val Thr Gly Ala Thr Cys Asn Gly Glu Ser Ser Asp
65                  70                  75                  80

Gly Phe Thr Phe Thr Pro Gly Asn Thr Val Ser Cys Val Val Gly Ser
                85                  90                  95

Thr Thr Ile Ala Thr Phe Asn Thr Gln Ser Glu Ala Ala Arg Ser Leu
            100                 105                 110

Arg Ala Val Asp Lys Val Ser Phe Ser Leu Glu Asp Ala Gln Glu Leu
        115                 120                 125

Ala Asn Ser Glu Asn Lys Lys Thr Asn Ala Ile Ser Leu Val Thr Ser
    130                 135                 140

Ser Asp Ser Cys Pro Ala Asp Ala Glu Gln Leu Cys Leu Thr Phe Ser
145                 150                 155                 160

Ser Val Val Asp Arg Ala Arg Phe Glu Lys Leu Tyr Lys Gln Ile Asp
                165                 170                 175

Leu Ala Thr Asp Asn Phe Ser Lys Leu Val Asn Glu Glu Val Glu Asn
            180                 185                 190

Asn Ala Ala Thr Asp Lys Ala Pro Ser Thr His Thr Ser Thr Val Val
        195                 200                 205

Pro Val Thr Thr Glu Gly Thr Lys Pro Asp Leu Asn Ala Ser Phe Val
    210                 215                 220

Ser Ala Asn Ala Glu Gln Phe Tyr Gln Tyr Gln Pro Thr Glu Ile Ile
225                 230                 235                 240

Leu Ser Glu Gly Gln Leu Val Asp Ser Leu Gly Asn Gly Val Ala Gly
                245                 250                 255

Val Asp Tyr Tyr Thr Asn Ser Gly Arg Gly Val Thr Asp Glu Asn Gly
            260                 265                 270

Lys Phe Ser Phe Ser Trp Gly Glu Thr Ile Ser Phe Gly Ile Asp Thr
        275                 280                 285

Phe Glu Leu Gly Ser Val Arg Gly Asn Lys Ser Thr Ile Ala Leu Thr
    290                 295                 300

Glu Leu Gly Asp Glu Val Arg Gly Ala Asn Ile Asp Gln Leu Ile His
305                 310                 315                 320

Arg Tyr Ser Thr Thr Gly Gln Asn Asn Thr Arg Val Val Pro Asp Asp
                325                 330                 335

Val Arg Lys Val Phe Ala Glu Tyr Pro Asn Val Ile Asn Glu Ile Ile
            340                 345                 350

Asn Leu Ser Leu Ser Asn Gly Ala Thr Leu Asp Glu Gly Asp Gln Asn
        355                 360                 365

Val Val Leu Pro Asn Glu Phe Ile Glu Gln Phe Lys Thr Gly Gln Ala
    370                 375                 380

Lys Glu Ile Asp Thr Ala Ile Cys Ala Lys Thr Asp Gly Cys Asn Glu
385                 390                 395                 400

Ala Arg Trp Phe Ser Leu Thr Thr Arg Asn Val Asn Asp Gly Gln Ile
                405                 410                 415

Gln Gly Val Ile Asn Lys Leu Trp Gly Val Asp Thr Asn Tyr Gln Ser
            420                 425                 430

Val Ser Lys Phe His Val Phe His Asp Ser Thr Asn Phe Tyr Gly Ser

435                 440                 445

Thr Gly Asn Ala Arg Gly Gln Ala Val Val Asn Ile Ser Asn Ser Ala
    450                 455                 460

Phe Pro Ile Leu Met Ala Arg Asn Asp Lys Asn Tyr Trp Leu Ala Phe
465                 470                 475                 480

Gly Glu Lys Arg Ala Trp Asp Lys Asn Glu Leu Ala Tyr Ile Thr Glu
                485                 490                 495

Ala Pro Ser Ile Val Gln Pro Glu Asn Val Thr Arg Asp Thr Ala Thr
            500                 505                 510

Phe Asn Leu Pro Phe Ile Ser Leu Gly Gln Val Gly Glu Gly Lys Leu
        515                 520                 525

Met Val Ile Gly Asn Pro His Tyr Asn Ser Ile Leu Arg Cys Pro Asn
    530                 535                 540

Gly Tyr Ser Trp Gly Gly Gly Val Asn Ser Lys Gly Glu Cys Thr Leu
545                 550                 555                 560

Ser Gly Asp Ser Asp Asp Met Lys His Phe Met Gln Asn Val Leu Arg
                565                 570                 575

Tyr Leu Ser Asn Asp Ile Trp Gln Pro Asn Thr Lys Ser Ile Met Thr
            580                 585                 590

Val Gly Thr Asn Leu Glu Asn Val Tyr Phe Lys Lys Ala Gly Gln Val
        595                 600                 605

Leu Gly Asn Ser Ala Pro Phe Ala Phe His Glu Asp Phe Thr Gly Ile
    610                 615                 620

Thr Val Lys Gln Leu Thr Ser Tyr Gly Asp Leu Asn Pro Glu Glu Ile
625                 630                 635                 640

Pro Leu Leu Ile Leu Asn Gly Phe Glu Tyr Val Thr Gln Trp Ser Gly
                645                 650                 655

Asp Pro Tyr Ala Val Pro Leu Arg Ala Asp Thr Ser Lys Pro Lys Leu
            660                 665                 670

Thr Gln Gln Asp Val Thr Asp Leu Ile Ala Tyr Leu Asn Lys Gly Gly
        675                 680                 685

Ser Val Leu Ile Met Glu Asn Val Met Ser Asn Leu Lys Glu Glu Ser
    690                 695                 700

Ala Ser Ser Phe Val Arg Leu Leu Asp Ala Ala Gly Leu Ser Met Ala
705                 710                 715                 720

Leu Asn Lys Ser Val Val Asn Asn Asp Pro Gln Gly Tyr Pro Asp Arg
                725                 730                 735

Val Arg Gln Arg Arg Ala Thr Gly Ile Trp Val Tyr Glu Arg Tyr Pro
            740                 745                 750

Ala Ala Asp Gly Ala Gln Pro Pro Tyr Thr Ile Asp Pro Asn Thr Gly
        755                 760                 765

Glu Val Thr Trp Lys Tyr Gln Gln Asp Asn Lys Pro Asp Asp Lys Pro
    770                 775                 780

Lys Leu Glu Val Ala Ser Trp Gln Glu Glu Val Glu Gly Lys Gln Val
785                 790                 795                 800

Thr Arg Tyr Ala Phe Ile Asp Glu Ala Glu Tyr Thr Thr Glu Glu Ser
                805                 810                 815

Leu Glu Ala Ala Lys Ala Lys Ile Phe Glu Lys Phe Pro Gly Leu Gln
            820                 825                 830

Glu Cys Lys Asp Ser Thr Tyr His Tyr Glu Ile Asn Cys Leu Glu Arg
        835                 840                 845

Arg Pro Gly Thr Asp Val Pro Val Thr Gly Gly Met Tyr Val Pro Arg
    850                 855                 860

Tyr Thr Gln Leu Asn Leu Asp Ala Asp Thr Ala Lys Ala Met Val Gln
865                 870                 875                 880

Ala Ala Asp Leu Gly Thr Asn Ile Gln Arg Leu Tyr Gln His Glu Leu
                885                 890                 895

Tyr Phe Arg Thr Lys Gly Ser Lys Gly Glu Arg Leu Asn Ser Val Asp
            900                 905                 910

Leu Glu Arg Leu Tyr Gln Asn Met Ser Val Trp Leu Trp Asn Asp Thr
        915                 920                 925

Lys Tyr Arg Tyr Glu Glu Gly Lys Glu Asp Glu Leu Gly Phe Lys Thr
    930                 935                 940

Phe Thr Glu Phe Leu Asn Cys Tyr Ala Asn Asp Ala Tyr Ala Gly Gly
945                 950                 955                 960

Thr Lys Cys Ser Ala Asp Leu Lys Lys Ser Leu Val Asp Asn Asn Met
                965                 970                 975

Ile Tyr Gly Asp Gly Ser Ser Lys Ala Gly Met Met Asn Pro Ser Tyr
            980                 985                 990

Pro Leu Asn Tyr Met Glu Lys Pro  Leu Thr Arg Leu Met  Leu Gly Arg
        995                 1000                 1005

Ser Trp  Trp Asp Leu Asn Ile  Lys Val Asp Val Glu  Lys Tyr Pro
    1010                 1015                 1020

Gly Ser  Val Ser Ala Lys Gly  Glu Ser Val Thr Glu  Asn Ile Ser
    1025                 1030                 1035

Leu Tyr  Ser Asn Pro Thr Lys  Trp Phe Ala Gly Asn  Met Gln Ser
    1040                 1045                 1050

Thr Gly  Leu Trp Ala Pro Ala  Gln Gln Asp Val Thr  Ile Lys Ser
    1055                 1060                 1065

Ser Ala  Ser Val Pro Val Thr  Val Thr Val Ala Leu  Ala Asp Asp
    1070                 1075                 1080

Leu Thr  Gly Arg Glu Lys His  Glu Val Ala Leu Asn  Arg Pro Pro
    1085                 1090                 1095

Arg Val  Thr Lys Thr Tyr Thr  Leu Glu Ala Asn Gly  Glu Val Thr
    1100                 1105                 1110

Phe Lys  Val Pro Tyr Gly Gly  Leu Ile Tyr Ile Lys  Gly Asp Ser
    1115                 1120                 1125

Lys Asp  Asp Val Ser Ala Asn  Phe Thr Phe Thr Gly  Val Val Lys
    1130                 1135                 1140

Ala Pro  Phe Tyr Lys Asp Gly  Glu Trp Lys Asn Asp  Leu Asp Ser
    1145                 1150                 1155

Pro Ala  Pro Leu Gly Glu Leu  Glu Ser Ala Ser Phe  Val Tyr Thr
    1160                 1165                 1170

Thr Pro  Lys Lys Asn Leu Glu  Ala Ser Asn Phe Thr  Gly Gly Val
    1175                 1180                 1185

Ala Glu Phe Ala Lys Asp Leu Asp Thr Phe Ala Ser Ser Met Asn
    1190                1195                1200

Asp Phe Tyr Gly Arg Asn Asp Glu Asp Gly Lys His Arg Met Phe
    1205                1210                1215

Thr Tyr Lys Asn Leu Thr Gly His Lys His Arg Phe Thr Asn Asp
    1220                1225                1230

Val Gln Ile Ser Ile Gly Asp Ala His Ser Gly Tyr Pro Val Met
    1235                1240                1245

Asn Ser Ser Phe Ser Thr Asn Ser Thr Thr Leu Pro Thr Thr Pro
    1250                1255                1260

Leu Asn Asp Trp Leu Ile Trp His Glu Val Gly His Asn Ala Ala
    1265                1270                1275

Glu Thr Pro Leu Asn Val Pro Gly Ala Thr Glu Val Ala Asn Asn
    1280                1285                1290

Val Leu Ala Leu Tyr Met Gln Asp Arg Tyr Leu Gly Lys Met Asn
    1295                1300                1305

Arg Val Ala Asp Asp Ile Thr Val Ala Pro Glu Tyr Leu Asp Glu
    1310                1315                1320

Ser Asn Gly Gln Ala Trp Ala Arg Gly Gly Ala Gly Asp Arg Leu
    1325                1330                1335

Leu Met Tyr Ala Gln Leu Lys Glu Trp Ala Glu Glu Asn Phe Asp
    1340                1345                1350

Ile Lys Gln Trp Tyr Pro Asp Gly Glu Leu Pro Lys Phe Tyr Ser
    1355                1360                1365

Asp Arg Lys Gly Met Lys Gly Trp Asn Leu Phe Gln Leu Met His
    1370                1375                1380

Arg Lys Ala Arg Gly Asp Asp Val Gly Asn Ser Thr Phe Gly Gly
    1385                1390                1395

Lys Asn Tyr Cys Ala Glu Ser Asn Gly Asn Ala Ala Asp Thr Leu
    1400                1405                1410

Met Leu Cys Ala Ser Trp Val Ala Gln Ala Asp Leu Ser Glu Phe

1415                    1420                    1425

Phe Lys  Lys Trp Asn Pro Gly  Ala Ser Ala Tyr Gln  Leu Pro Gly
    1430                    1435                    1440

Ala Thr  Glu Met Ser Phe Gln  Gly Gly Val Ser Ser  Ser Ala Tyr
    1445                    1450                    1455

Ser Thr  Leu Ala Ser Leu Lys  Leu Pro Lys Pro Glu  Lys Gly Pro
    1460                    1465                    1470

Glu Thr  Ile Asn Lys Val Thr  Glu His Lys Met Ser  Ala Glu
    1475                    1480                    1485

<210>  16
<211>  414
<212>  PRT
<213>  E.coli

<400>  16

Val Ala Asp Gly Gln Gln Ala Tyr Thr Leu Thr Leu Thr Ala Val Asp
1               5                   10                  15

Ser Glu Gly Asn Pro Val Thr Gly Glu Ala Ser Arg Leu Arg Leu Val
            20                  25                  30

Pro Gln Asp Thr Asn Gly Val Thr Val Gly Ala Ile Ser Glu Ile Lys
        35                  40                  45

Pro Gly Val Tyr Ser Ala Thr Val Ser Ser Thr Arg Ala Gly Asn Val
    50                  55                  60

Val Val Arg Ala Phe Ser Glu Gln Tyr Gln Leu Gly Thr Leu Gln Gln
65                  70                  75                  80

Thr Leu Lys Phe Val Ala Gly Pro Leu Asp Ala Ala His Ser Ser Ile
                85                  90                  95

Thr Leu Asn Pro Asp Lys Pro Val Val Gly Gly Thr Val Thr Ala Ile
            100                 105                 110

Trp Thr Ala Lys Asp Ala Asn Asp Asn Pro Val Thr Gly Leu Asn Pro
        115                 120                 125

Asp Ala Pro Ser Leu Ser Gly Ala Ala Ala Ala Gly Ser Thr Ala Ser
    130                 135                 140

Gly Trp Thr Asp Asn Gly Asp Gly Thr Trp Thr Ala Gln Ile Ser Leu

145                 150                 155                 160

Gly Thr Thr Ala Gly Glu Leu Asp Val Met Pro Lys Leu Asn Gly Gln
                165                 170                 175

Asp Ala Ala Ala Asn Ala Ala Lys Val Thr Val Val Ala Asp Ala Leu
            180                 185                 190

Ser Ser Asn Gln Ser Lys Val Ser Val Ala Glu Asp His Val Lys Ala
        195                 200                 205

Gly Glu Ser Thr Thr Val Thr Leu Val Ala Lys Asp Ala His Gly Asn
    210                 215                 220

Ala Ile Ser Gly Leu Ser Leu Ser Ala Ser Leu Thr Gly Thr Ala Ser
225                 230                 235                 240

Glu Gly Ala Thr Val Ser Ser Trp Thr Glu Lys Gly Asp Gly Ser Tyr
                245                 250                 255

Val Ala Thr Leu Thr Thr Gly Gly Lys Thr Gly Glu Leu Arg Val Met
            260                 265                 270

Pro Leu Phe Asn Gly Gln Pro Ala Ala Thr Glu Ala Ala Gln Leu Thr
        275                 280                 285

Val Ile Ala Gly Glu Met Ser Ser Ala Asn Ser Thr Leu Val Ala Asp
    290                 295                 300

Asn Lys Thr Pro Thr Val Lys Thr Thr Thr Glu Leu Thr Phe Thr Met
305                 310                 315                 320

Lys Asp Ala Tyr Gly Asn Pro Val Thr Gly Leu Lys Pro Asp Ala Pro
                325                 330                 335

Val Phe Ser Gly Ala Ala Ser Thr Gly Ser Glu Arg Pro Ser Ala Gly
            340                 345                 350

Asn Trp Thr Glu Lys Gly Asn Gly Val Tyr Val Ser Thr Leu Thr Leu
        355                 360                 365

Gly Ser Ala Ala Gly Gln Leu Ser Val Met Pro Arg Val Asn Gly Gln
    370                 375                 380

Asn Ala Val Ala Gln Pro Leu Val Leu Asn Val Ala Gly Asp Ala Ser
385                 390                 395                 400

Lys Ala Glu Ile Arg Asp Met Thr Val Lys Val Asn Asn Gln
                405                 410